# EUROPEAN PATENT SPECIFICATION

(11) **EP 4 301 383 B1**
(45) Date of publication and mention of the grant of the patent: **23.07.2025**
(21) Application number: 22707500.9
(22) Date of filing: 25.02.2022
(51) Int. Cl.: A61K 35/614, A61K 8/64, A61K 9/00, A61K 9/06, A61K 45/06, A61P 17/00

(54) **COLLAGENOUS EXTRACTS FOR USE AS A MEDICAMENT**
KOLLAGENEXTRAKTE ZUR VERWENDUNG ALS ARZNEIMITTEL
EXTRAITS DE COLLAGÈNE DESTINÉS À ÊTRE UTILISÉS EN TANT QUE MÉDICAMENT

(30) Priority: 04.03.2021 GB 202103046
(43) Date of publication of application: 10.01.2024
(73) Proprietor: Jellagen Limited, Cardiff CF3 2PY (GB)
(72) Inventor: SPRAGG, Andrew Mearns, Cardiff CF3 2PY (GB); BARNARD, Almero, Cardiff CF3 2PY (GB)
(74) Representative: Potter Clarkson
(86) International application number: PCT/GB2022/050521
(87) International publication number: WO 2022/185033

(56) References cited:
- WO-A1-2011/140526
- WO-A1-2012/149136
- US-A1- 2004 167 318
- US-A1- 2005 271 614
- JIN-FENG DING ET AL: "Study on effect of jellyfish collagen hydrolysate on anti-fatigue and anti-oxidation", FOOD HYDROCOLLOIDS, ELSEVIER BV, NL, vol. 25, no. 5, 23 December 2010 (2010-12-23), pages 1350 - 1353, XP028163286, ISSN: 0268-005X, [retrieved on 20110104], DOI: 10.1016/J.FOODHYD.2010.12.013
- WOODLEY D T ET AL: "Injection of recombinant human type VII collagen restores collagen function in dystrophic epidermolysis bullosa", NATURE MEDICINE, NATURE PUBLISHING GROUP US, NEW YORK, vol. 10, no. 7, 1 July 2004 (2004-07-01), pages 693 - 695, XP008108910, ISSN: 1078-8956, [retrieved on 20040613], DOI: 10.1038/NM1063
- YONGLIANG ZHUANG ET AL: "Effects of Collagen and Collagen Hydrolysate from Jellyfish (Rhopilema esculentum) on Mice Skin Photoaging Induced by UV Irradiation", JOURNAL OF FOOD SCIENCE, vol. 74, no. 6, 1 August 2009 (2009-08-01), US, pages H183 - H188, XP055402206, ISSN: 0022-1147, DOI: 10.1111/j.1750-3841.2009.01236.x

## Description

### FIELD OF THE INVENTION

The present invention relates to a collagenous marine invertebrate extract for use in the treatment of Epidermolysis Bullosa, pharmaceutical compositions comprising said collagenous marine invertebrate extract, and methods for manufacturing the collagenous marine invertebrate extract of the invention.

### BACKGROUND

Epidermolysis bullosa (EB) is a group of genetic skin diseases that cause the skin to blister and erode very easily. In people with EB, blisters form in response to minor injuries or friction, such as rubbing or scratching. In most cases, the onset of EB is at birth or shortly after. It is estimated that half a million people worldwide are affected by the disease, many of whom are children.

Human skin consists of two layers: an outermost layer called the epidermis and a layer underneath called the dermis. In individuals with healthy skin, there exist protein anchors between these two layers that prevent them from moving independently from one another (shearing). In people born with EB, the two skin layers lack the protein anchors that hold them together, resulting in extremely fragile skin.

The disease severity can range from mild to fatal. Complications may include oesophageal narrowing, and the need for amputations. The most potentially dangerous complication of EB is Squamous Cell Carcinoma (SCC). Unfortunately, SCC is often invasive and metastasises rapidly in patients with EB. Moreover, there is no clear consensus regarding the best treatment strategies. There are five main types of EB, which are classified based on the depth or level of blister formation:

### 1. Epidermolysis bullosa simplex (EBS)

Blistering occurs in the upper layer of the skin, called the epidermis. It is characterised by a lack of adhesion of the skin directly above the basement membrane (the basal layer). EBS is the most common type of EB, accounting for around 55 % of cases. EBS is generally milder than other types of EB, although the blistering is painful and easily compounded by rubbing. Genes associated with EBS include: K₅/KRT5 (keratin-5); K₁₄ (keratin-14); TGM5 (transglutaminase-5); DSP (desmoplakin); PKP1 (plakophilin-1); PLEC (plectin); DST (dystonin); ITGA6 (α6B4 Integrin 1); and, ITGR4 ( α6B4 Integrin); COL17A1 (collagen type XVII); and, JUP (junction plakoglobin).

### 2. Junctional epidermolysis bullosa (JEB)

Blistering occurs in a skin layer called the lamina lucida within the basement membrane zone, which is situated at the junction between the epidermis (upper layer of the skin) and the dermis (lower layer). JEB is the most severe type of EB and accounts for about 5 % of cases. Genes associated with JEB include: LAMA3 (laminin-332); LAMB3 (laminin-332); LAMC2 (laminin-332); COL17A1 (collagen type XVII); ITGA6 (α6B4 Integrin 1); and, ITGR4 ( α6B4 Integrin).

### 3. Dystrophic epidermolysis bullosa (DEB)

Tissue separation and blistering occur in the basement membrane due to problems in attachment between the epidermis and dermis. The mode of inheritance may be dominant (DDEB) or recessive (RDEB) but all forms of DEB result from mutations in the gene encoding the anchoring fibril protein, type VII collagen, a structural protein vital for the elastic and structural integrity of the skin. DEB is a particularly severe form of EB. Squamous Cell CarcinomaI (SCC) is by far the greatest cause for mortality in DEB patients with 80 % succumbing to SCC by age 55. DEB accounts for about 30% of cases. In RDEB, certain genes (and corresponding proteins) are upregulated suggesting that MMP-3 may play an important role in the blister formation of the skin by the degradation of anchoring fibrils consisting of type VII collagen (D. Sawamura et al., 1991. Biochem Biophys Res Commun., 174(2):1003-8..

### 4. Epidermolysis bullosa acquisita (EBA)

EBA is a non-genetic autoimmune disease, caused by the development of antibodies against type VII collagen. An examination of skin blisters will show antibodies deposited in the basement membrane between the epidermis and dermis. This form of EB is very rare, is not inherited, and usually develops during adulthood.

### 5. Kindler syndrome (KS)

Blistering may occur at multiple levels within the basement membrane zone, or in skin layers beneath it. This syndrome is rare within this disease, and characterised by blisters on the hands and feet, altered skin colouring, and damage to the inner lining of areas such as the mouth, intestines, or eyes. Genes associated with KS include FERMT1 (kindlin-1).

DEB in particular is caused by mutations in the COL7A1 gene. Mutations in COL7A1 lead to defects in a protein called type VII collagen (C7), which is crucial for the proper bonding of the two skin layers, the epidermis and the dermis. Type VII collagen (C7) is a collagen found in the skin of humans and other animals. It is localised in the basement membrane zone (BMZ) between the epidermis and dermis in large structures called Anchoring Fibrils (AFs), which are composed of C7. The AFs hold the epidermis and dermis together.

Today, there is no cure for the condition with disease management comprising of mostly supportive wound care, pain control, controlling infections, nutritional support, and prevention and treatment of complications. Monitoring for complications with laboratory testing and imaging studies is also important, although the frequency of these tests will vary depending on the type of EB and severity in each person. Managing EB requires a multidisciplinary team of health care providers including a dermatologist, EB nurse who specialises in wound care, occupational therapist, nutritionist, and social worker. Various other specialists may be consulted as needed. Disease management is individualised for each person depending on their age, severity, symptoms, complications, and priorities.

Various attempts at utilising collagen for EB have resulted in a lack of success. For example, an investigational therapy known as PTR-01 collagen is attempting to use Recombinant type VII (rC7) for the treatment of Recessive Dystrophic Epidermolysis Bullosa. rC7 is a potentially disease-modifying drug that is delivered intravenously to patients, replacing defective collagen type VII with healthy collagen at the sites where it is needed. Although preclinical studies have shown promising results with recombinant collagen type VII in animals, topical rC7 gel *in vivo* can only be used on open lesions because it does not penetrate intact skin. Additionally, intravenous (IV) rC7 does not appear to be appropriate for EB. In its intravenous formulation, it seems that rC7 generates some toxicity issues.

There are a myriad of disadvantages associated with using this type of collagen. For instance, purified forms of human C7 can only be extracted only in very small quantities from human skin, amnion, and skin cells such as keratinocytes or dermal fibroblasts. However, large amounts of C7 cannot be generated from such sources in this manner. Furthermore, it is also well known that the use of mammalian collagen (bovine and porcine) in tissue engineering is associated with considerable risk of disease and virus transmission and the purification of collagen from mammalian sources is associated with considerable expense.

Thus, there is a need for an efficient treatment for EB, that overcomes these disadvantages.

Surprisingly, the applicant has identified that a collagenous extract derived from marine invertebrates lacks the disadvantages described above, and offers an opportunity for the treatment of EB. This invention is based on the entirely unexpected finding that, despite the vastly different physicochemical properties of collagenous marine invertebrate extracts in comparison to mammalian-derived or fish-derived collagen, heat-treated collagenous marine invertebrate extracts (unlike heat treated collagenous extracts from mammals) can be used for therapeutic gene modulation useful in the treatment of EB. Specifically, heat-treated collagenous extracts derived from marine invertebrates are able to upregulate the expression of key genes that are faulty in EB leading to the increased expression of anchoring proteins in EB diseased skin.

### DESCRIPTION OF THE INVENTION

In a first aspect of the invention, there is provided a collagenous marine invertebrate extract for use in the treatment of Epidermolysis Bullosa.

Marine invertebrates refer to the invertebrates animals that live in marine habitats. They make up most of the macroscopic life in the oceans and have a large variety of body plans. They can be categorised into over 30 phyla, including Acoela, Annelida, (polychaetes and sea leeches), Brachiopoda (marine animals that have hard "valves" (shells) on the upper and lower surfaces), Bryozoa (also known as moss animals or sea mats), Chaetognatha (commonly known as arrow worms), Cephalochordata (represented in the modern oceans by the lancelets), Cnidaria (such as jellyfish, sea anemones, and corals), Crustacea (including lobsters, crabs, shrimp, crayfish, barnacles, hermit crabs, mantis shrimps, and copepods), Ctenophora (also known as comb jellies, the largest animals that swim by means of cilia), Echinodermata (including sea stars, brittle stars, sea urchins, sand dollars, sea cucumbers, crinoids, and sea daisies), Echiura (spoon worms), Gnathostomulids (slender to thread-like worms, with a transparent body that inhabit sand and mud beneath shallow coastal waters), Gastrotricha (often called hairy backs, found mostly interstitially in between sediment particles), Hemichordata (includes acorn worms, solitary worm-shaped organisms), Kamptozoa (goblet-shaped sessile aquatic animals, with relatively long stalks and a "crown" of solid tentacles, also called Entoprocta), Kinorhyncha (segmented, limbless animals, widespread in mud or sand at all depths, also called mud dragons), Loricifera (very small to microscopic marine sediment-dwelling animals), Mollusca (including shellfish, squid, octopus, whelks, Nautilus, cuttlefish, nudibranchs, scallops, sea snails, Aplacophora, Caudofoveata, Monoplacophora, Polyplacophora, and Scaphopoda), Myzostomida (small marine worms which are parasitic on crinoids or "sea lilies"), Nemertinea (also known as "ribbon worms" or "proboscis worms"), Orthonectida (among the simplest of multi-cellular organisms), Phoronida (marine animals that filter-feed with a lophophore (a "crown" of tentacles and build upright tubes of chitin to support and protect their soft bodies), Placozoa, (small, flattened, multicellular animals around 1 millimetre across and the simplest in structure), Porifera (sponges, multicellular organisms that have bodies full of pores and channels allowing water to circulate through them), Priapulida (marine worms that live marine mud), Pycnogonida (also called sea spiders), Sipunculida (also called peanut worms, is a group of bilaterally symmetrical, unsegmented marine worms), Tunicata (also known as sea squirts or sea pork, are filter feeders attached to rocks or similarly suitable surfaces on the ocean floor), some flatworms of the classes Turbellaria and Monogenea, Xenoturbella (bilaterian animals that contains only two marine worm-like species), Xiphosura (including a large number of extinct lineages and only four recent species in the family Limulidae, which include the horseshoe crabs).

Preferably, the collagenous marine invertebrate extract is from Cnidaria, more preferably from jellyfish.

"Cnidaria" refers to the exclusively aquatic phylum represented by polyps such as sea anemones and corals, and by medusae such as jellyfish. A polypoid or a medusoid cnidarian is a radially or biradially symmetrical, uncephalised animal with a single body opening, the mouth.

"Jellyfish" refers to the medusa-phase of gelatinous members of the subphylum Medusozoa, a major part of the phylum Cnidaria. Jellyfish are mainly free-swimming marine gelatinous zooplanktonic organism with umbrella-shaped bells and trailing tentacles, although a few are not mobile, being anchored to the seabed by stalks. The bell can pulsate to provide propulsion and highly efficient locomotion. The tentacles are armed with stinging cells and may be used to capture prey and defend against predators. Jellyfish have a complex life cycle: the medusa is normally the sexual phase; the planula larva can disperse widely and is followed by a sedentary polyp phase.

The jellyfish may be from the class *Scyphozoa.* Preferably, the jellyfish is one or more selected from the group comprising: *Rhizostomas pulmo, Rhopilema esculentum, Rhopilema nomadica, Stomolophus meleagris, Aurelia sp., Nemopilema nomurai, Cassiopea andromeda, Nemopilema nomurai, Chrysaora sp.,* or any combination thereof

"*Rhizostomas pulmo*", commonly known as the barrel jellyfish, is a scyphomedusa in the family *Rhizostomatidae.* It is found in the northeast and in the southern Atlantic, in the Adriatic, Mediterranean Sea, Black Sea and Sea of Azov. It typically is up to 40 cm in diameter, and is a large blue jellyfish, whose parasol can measure up to one meter. It has four arms that are divided into eight welded arms at the manubrium. It does not have tentacles around the umbrella. Small fish, such as boops, seriolas and trachurus, take shelter under its umbrella or between its arms. The extremities of its eight arms contain symbiotic algae: zooxanthellae, which in exchange for housing and light produce food whose unused excess is consumed by the jellyfish. *R. pulmo* collagen consists of two chains 1α and 2α and contain a heparin binding site that would not be located in the triple helix but rather in the linear portion of the protein.

"*Rhopilema esculentum*", the flame jellyfish, is a species of jellyfish native to the warm temperate waters of the Pacific Ocean. Like other members of the order *Rhizostomae*, the medusa stage of *Rhopilema esculentum* has no tentacles at the margin of the bell. Instead, underneath the bell it has eight highly branched oral arms, fused at the base and with numerous secondary mouth openings.

"*Rhopilema nomadica*" is a jellyfish indigenous to tropical warm waters of the Indian and Pacific Oceans. Its body is light blue and the bell is rounded. It can grow up to 10 kg of weight, and its bell is commonly 40-60 cm in diameter, but can be up to 90 cm.

"*Stomolophus meleagris*" or the cannonball jellyfish, is most abundant in the south eastern United States and the Gulf Coast. This is a small jellyfish, with a height of 12.7 cm and a width of 18.0 cm. This jellyfish has 16 short, forked fused orals arms instead of the normal tentacles. *Stomolophus meleagris* also has secondary mouth folds (scapulets) covered with mucus, thought to be for trapping small prey.

"*Aurelia sp*." or *aurelia aurita* is a widely studied species of the genus *Aurelia.* This jellyfish is translucent, usually about 25-40 cm (10-16 in) in diameter, and can be recognised by its four horseshoe-shaped gonads, easily seen through the top of the bell. It feeds by collecting medusae, plankton, and mollusks with its tentacles, and bringing them into its body for digestion. It is capable of only limited motion, and drifts with the current, even when swimming.

"*Nemopilema nomurai*" is a giant jellyfish reaching 2 m and 220 kg, which can be found in the Sea of Japan. The body color is translucent bluish and the underlying tentacles are yellowish in young, brownish individuals when they are older.

"*Cassiopea andromeda*" is one of many cnidarian species called the upside-down jellyfish. It usually lives in intertidal sand or mudflats, shallow lagoons, and around mangroves. This jellyfish, often mistaken for a sea anemone, usually keeps its mouth facing upward.

Its yellow-brown bell, which has white or pale streaks and spots, pulsates to run water through its arms for respiration and to gather food.

More preferably, said jellyfish is *Rhizostomas pulmo.*

Collagen is one of the most abundant proteins in the extracellular matrix of animal bodies. The central feature of all collagen molecules is their stiff, triple-stranded helical structure. Types I and III are the main types of collagen found in connective tissue and constitute 90% of all collagen. Besides its role in mechanical strength, collagen functions as a signalling molecule, regulating cellular migration, differentiation (Bosnakovski et al, 2005. Cell Tissue Res., 319(2):243-53) and proliferation (Pozzi et al, 1998. J. Cell Biol., 142(2):587-94), and functions in haemostasis (Farndale et al, 2004. J. Thromb. Haemost., 2(4):561-73).

Preferably, the collagenous marine invertebrate extract is for use in the treatment of Epidermolysis bullosa simplex (EBS), Junctional epidermolysis bullosa (JEB), Dystrophic epidermolysis bullosa (DEB), Epidermolysis bullosa acquisita (EBA), and/or Kindler syndrome (KS).

Preferably, the collagenous marine invertebrate extract is for use in the treatment of Dystrophic epidermolysis bullosa (DEB).

In one embodiment, the collagenous marine invertebrate extract:
(a) supports improved dermal cohesion and basal layer structuring of cells via the activation of the genes COL7A1, LAMA5, KRT5;
(b) downregulates MMP-3 activity supporting better treatment outcomes for severe RDEB;
(c) supports improved cell adhesion, growth, migration, and differentiation via the activation of the genes FN1;
(d) promotes extracellular matrix synthesis, collagen fibre organisation and structuring via activation of the genes COL3A1, FBN1, FBN2, SPARC, and LUM;
(e) supports improved dermal cohesion and basal layer structuring of cells via the activation of the genes COL7A1, LAMA5 and CDC42;
(f) supports dermis renewal and protection via the activation of Epithelial Growth Factor (EGF), SIRT4 and SIRT6;
(g) supports improved dermal cellular junction structure via activation of the genes EPPK and LOR, and/or
(h) supports improved extracellular matrix structure by reducing MMP3 activation involved in EB blistering.

"Extracellular matrix" refers to the three-dimensional network of extracellular macromolecules, such as collagen, enzymes, and glycoproteins, that provide structural and biochemical support of surrounding cells (Theocharis AD et al. 2016. Adv. Drug Deliv. Rev., 97: 4-27). The composition of ECM varies between multicellular structures. However, cell adhesion, cell-to-cell communication and differentiation are common functions of the ECM (Abedin and King. 2010. Trends Cell Biol., 20(12): 734-42).

In one embodiment, the collagenous marine invertebrate extract is for use in supporting improved dermal-epidermal junction (DEJ) cohesion and basal layer structuring of cells via the activation of the genes COL7A1, COL17A1, DSP, ITGA6, ITGB4, LAMB3, LAMC2, LOX, FBN1, FBN2, FN1 and KRT5. Furthermore, said hydrolysed collagenous jellyfish extract reduces the expression of gene MMP3.

"Dermal-Epidermal Junction" refers to the interface between basal layer keratinocytes of the epidermis and the dermis and helps the skin resist shearing forces. The DEJ is comprised of hemidesmosomes, anchoring filaments, anchoring fibrils, and type VII collagen, which combine to tether the dermis to the epidermis.

"Activation" means that the collagenous marine invertebrate extract according to the invention stimulates the transcription of a gene of interest and/or the expression of the proteins encoded by such genes.

CDC42 gene encodes a small GTPase protein of the Rho-subfamily, which regulates signalling pathways that control diverse cellular functions including cell morphology, migration, endocytosis and cell cycle progression. This protein is able to complement the yeast cdc42-1 mutant. The product of oncogene Dbl was reported to specifically catalyse the dissociation of GDP from this protein. This protein could regulate actin polymerisation through its direct binding to Neural Wiskott-Aldrich syndrome protein (N-WASP), which subsequently activates Arp2/3 complex. Alternative splicing of this gene results in multiple transcript variants. Pseudogenes of this gene have been identified on chromosomes 3, 4, 5, 7, 8 and 20.

COL3A1 is the gene coding for the type III collagen, which is the collagen of granulation tissue and which is produced quickly by young fibroblasts before the tougher type I collagen is synthesised. It is also found in reticular fibre, in artery walls, skin, intestines and the uterus.

COL7A1 gene provides instructions for making proteins that are used to assemble type VII collagen. Type VII collagen plays an essential role in strengthening and stabilising the skin. The proteins produced from the COL7A1 gene, called pro-α1(VII) chains, are the components of type VII collagen. Three pro-α1(VII) chains twist together to form a triple-stranded, rope-like molecule known as a procollagen. Procollagen molecules are secreted by the cell and processed by enzymes to remove extra protein segments from the ends. Once these molecules are processed, they arrange themselves into long, thin bundles of mature type VII collagen. Type VII collagen is the major component of structures in the skin called anchoring fibrils. These fibrils are found in a region known as the epidermal basement membrane zone, which is a two-layer membrane located between the top layer of skin, called the epidermis, and an underlying layer called the dermis. Anchoring fibrils hold the two layers of skin together by connecting the epidermal basement membrane to the dermis.

COL17A1 gene provides instructions for making proteins that are used to assemble type XVII collagen. Type XVII collagen s a transmembrane protein which plays a critical role in maintaining the linkage between the intracellular and the extracellular structural elements involved in epidermal adhesion. COL17A1 is the official name of the gene. It encodes the alpha chain of type XVII collagen. Collagen XVII is a transmembrane protein, like collagen XIII, XXIII and XXV. Collagen XVII is a structural component of hemidesmosomes, multiprotein complexes at the dermal-epidermal basement membrane zone that mediate adhesion of keratinocytes to the underlying membrane. It also appears to be a key protein in maintaining the integrity of the corneal epithelium. Mutations in this gene are associated with both generalised atrophic benign and junctional epidermolysis bullosa, as well as recurrent corneal erosions, and expression of this gene is abnormal in various cancers.

DSP gene encodes for the assembly of Desmoplakin. Desmoplakin is a critical component of desmosome structures in cardiac muscle and epidermal cells, which function to maintain the structural integrity at adjacent cell contacts.

Epithelial Growth Factor (EGF) gene encodes a member of the epidermal growth factor superfamily. The encoded preproprotein is proteolytically processed to generate the 53-amino acid epidermal growth factor peptide. This protein acts a potent mitogenic factor that plays an important role in the growth, proliferation and differentiation of numerous cell types. This protein acts by binding with high affinity to the cell surface receptor, epidermal growth factor receptor. Alternative splicing results in multiple transcript variants, at least one of which encodes a preproprotein that is proteolytically processed.

EPPK or KRT9 gene encodes the type I keratin 9, an intermediate filament chain expressed only in the terminally differentiated epidermis of palms and soles.

FBN1 is the gene coding for fibrillin 1, which is the main component of the microfibrils of the extracellular matrix of the dermis and participates in the formation of elastic fibres. It also plays a role in the regulation of different cytokines and growth factors and is found in both elastic and non-elastic connective tissue.

FBN2 is the gene coding for fibrillin 2, which is transported out of cells into the extracellular matrix. In the extracellular matrix, fibrillin-2 binds to other proteins to form thread-like filaments called microfibrils. Microfibrils become part of elastic fibres which enable the skin, ligaments, and blood vessels to stretch. Researchers have suggested that fibrillin-2 plays a role in directing the assembly of elastic fibres during embryonic development. Microfibrils also contribute to more rigid tissues that support the lens of the eye, nerves, and muscles. Additionally, microfibrils hold certain growth factors called transforming growth factor-beta (TGF-beta) proteins, which keeps them inactive. When released from microfibrils, TGF-beta growth factors are activated and affect the growth and repair of tissues throughout the body.

FN1 encodes Fibronectin 1. Fibronectin is an extracellular matrix protein important in wound healing.

ITGA6 gene encodes for the assembly of the ITGA6 protein product. Integrins are integral cell-surface proteins composed of an alpha chain and a beta chain. A given chain may combine with multiple partners resulting in different integrins. For example, alpha 6 may combine with beta 4 in the integrin referred to as TSP180, or with beta 1 in the integrin VLA-6. Integrins are known to participate in cell adhesion as well as cell-surface mediated signalling. Two transcript variants encoding different isoforms have been found for this gene. Specific loss of this integrin chain in the intestinal epithelium, and thus of their hemidesmosomes, induces long-standing colitis and infiltrating adenocarcinomas
ITGB4 gene encodes for the ITGB4 protein sub-unit of product. Integrins are heterodimers composed of alpha and beta subunits, that are noncovalently associated transmembrane glycoprotein receptors. Different combinations of alpha and beta polypeptides form complexes that vary in their ligand-binding specificities. Integrins mediate cell-matrix or cell-cell adhesion, and transduced signals that regulate gene expression and cell growth. This gene encodes the integrin beta 4 subunit, a receptor for the laminins. This subunit tends to associate with alpha 6 subunit and is likely to play a pivotal role in the biology of invasive carcinoma. Mutations in this gene are associated with epidermolysis bullosa with pyloric atresia. Multiple alternatively spliced transcript variants encoding distinct isoforms have been found for this gene

KRT5 gene encodes for the protein Keratin-5. This protein dimerises with keratin 14 and forms the intermediate filaments (IF) that make up the cytoskeleton of basal epithelial cells. This protein is involved in several diseases including epidermolysis bullosa simplex and breast and lung cancers
LAMA5 gene encodes one of the vertebrate laminin alpha chains. Laminins, a family of extracellular matrix glycoproteins, are the major non-collagenous constituent of basement membranes. They have been implicated in a wide variety of biological processes including cell adhesion, differentiation, migration, signalling, neurite outgrowth and metastasis. Laminins are composed of 3 non identical chains: laminin alpha, beta and gamma (formerly A, B1, and B2, respectively) and they form a cruciform structure consisting of 3 short arms, each formed by a different chain, and a long arm composed of all 3 chains. Each laminin chain is a multidomain protein encoded by a distinct gene. The protein encoded by this gene is the alpha-5 subunit of laminin-10 (laminin-511), laminin-11 (laminin-521) and laminin-15 (laminin-523).

LOR gene encodes loricrin, a major protein component of the cornified cell envelope found in terminally differentiated epidermal cells.

LOX gene encodes for Lysyl oxidases. LOX enzymes are important in the remodelling of the extracellular matrix (ECM) in animals by cross-linking collagens and elastin.

LUM is the gene coding for Lumican, which is an extracellular matrix protein of the small leucine-rich proteoglycan (SLRP) family. This protein is a major keratan sulfate proteoglycan of the cornea but is ubiquitously distributed in most mesenchymal tissues throughout the body. Lumican is involved in collagen fibril organisation and circumferential growth, corneal transparency, and epithelial cell migration and tissue repair. Corneal transparency is possible due to the exact alignment of collagen fibres by lumican (and keratocan) in the intrafibrillar space.

MMP3 is the gene coding for Stromelysin-1, also known as matrix metalloproteinase-3. Proteins of the matrix metalloproteinase (MMP) family are involved in the breakdown of extracellular matrix in normal physiological processes, such as embryonic development, reproduction, and tissue remodelling, as well as in disease processes, such as arthritis and metastasis. Most MMP's are secreted as inactive proproteins which are activated when cleaved by extracellular proteinases. Stromelysin-1 degrades fibronectin, laminin, collagens III, IV, IX, and X, and cartilage proteoglycans.

SIRT4 gene encodes a member of the sirtuin family of proteins. Members of the sirtuin family are characterised by a sirtuin core domain and grouped into four classes. Studies suggest that the human sirtuin may function as intracellular regulatory proteins with mono-ADP-ribosyltransferase activity. The protein encoded by this gene is included in class IV of the sirtuin family.

SIRT6 gene encodes a member of the sirtuin family of NAD-dependent enzymes that are implicated in cellular stress resistance, genomic stability, aging and energy homeostasis. The encoded protein is localised to the nucleus, exhibits ADP-ribosyl transferase and histone deacetylase activities, and plays a role in DNA repair, maintenance of telomeric chromatin, inflammation, lipid and glucose metabolism. Alternative splicing results in multiple transcript variants encoding different isoforms.

SPARC is the gene coding for Osteonectin or "cysteine-rich acidic matrix-associated protein". This protein is required for the collagen in bone to become calcified but is also involved in extracellular matrix synthesis and promotion of changes to cell shape. The gene product has been associated with tumour suppression but has also been correlated with metastasis based on changes to cell shape which can promote tumour cell invasion. Three transcript variants encoding different isoforms have been found for this gene.

TNFα gene encodes a multifunctional proinflammatory cytokine that belongs to the tumour necrosis factor (TNF) superfamily. This cytokine is mainly secreted by macrophages. It can bind to, and thus functions through its receptors TNFRSF1A/TNFR1 and TNFRSF1B/TNFBR. This cytokine is involved in the regulation of a wide spectrum of biological processes including cell proliferation, differentiation, apoptosis, lipid metabolism, and coagulation.

"Dermis" is the lower or inner layer of the two main layers of cells that make up the skin. "Dermis" or corium is a layer of skin between the epidermis (with which it makes up the cutis) and subcutaneous tissues, that primarily consists of dense irregular connective tissue and cushions the body from stress and strain. It is divided into two layers, the superficial area adjacent to the epidermis called the papillary region and a deep thicker area known as the reticular dermis. The dermis is tightly connected to the epidermis through a basement membrane. Structural components of the dermis are collagen, elastic fibres, and extrafibrillar matrix (Pengfei Lu et. al., 2011. Cold Spring Harb Perspect Biol. Dec; 3(12)). It also contains mechanoreceptors that provide the sense of touch and thermoreceptors that provide the sense of heat. In addition, hair follicles, sweat glands, sebaceous glands (oil glands), apocrine glands, lymphatic vessels, nerves and blood vessels are present in the dermis. Those blood vessels provide nourishment and waste removal for both dermal and epidermal cells.

"Basal layer" or stratum basale is the deepest layer of the five layers of the epidermis. This is a single layer of columnar or cuboidal cells. The cells are attached to each other and to the overlying stratum spinosum cells by desmosomes and hemidesmosomes. The nucleus is large, ovoid and occupies most of the cell. Some basal cells can act like stem cells with ability to divide and produced new cells, whereas others serve to anchor the epidermis glabrous skin (hairless), and hyper-proliferative epidermis (from a skin disease) (McGrath JA et al. (2004). Rook's Textbook of Dermatology (7th ed.). Blackwell Publishing. p. 4190.)

"Cellular junction structure" consists of multiprotein complexes that provide contact between neighbouring cells or between a cell and the extracellular matrix. They also build up the paracellular barrier of epithelia and control the paracellular transport. They are especially abundant in epithelial tissues.

"Inflammation" is part of the complex biological response of body tissues to harmful stimuli, such as pathogens, damaged cells, or irritants and is a protective response involving immune cells, blood vessels, and molecular mediators. The function of inflammation is to eliminate the initial cause of cell injury, clear out necrotic cells and tissues damaged from the original insult and the inflammatory process, and initiate tissue repair.

"Sensitive skin" refers to a skin condition in which skin is prone to itching and irritation.

TNFα gene encodes a multifunctional proinflammatory cytokine that belongs to the tumour necrosis factor (TNF) superfamily. This cytokine is mainly secreted by macrophages. It can bind to, and thus functions through its receptors TNFRSF1A/TNFR1 and TNFRSF1B/TNFBR. This cytokine is involved in the regulation of a wide spectrum of biological processes including cell proliferation, differentiation, apoptosis, lipid metabolism, and coagulation.

In one embodiment, the collagenous marine invertebrate extract comprises collagen in a hydrolysate form.

"Hydrolysate form" refers to a product resulting from any chemical reaction in which a molecule of water ruptures one or more chemical bonds (i.e. hydrolysis). This term covers substitution, elimination, and fragmentation reactions in which water is the nucleophile.

Hydrolysis of the collagenous extract and the collagen therein may be carried out using any method known in the art, such as through the action of proteolytic enzymes or by heat treatment. Preferably, the collagen in hydrolysate form is produced by heat treatment of the collagenous extract.

In one embodiment, the collagenous marine invertebrate extract is in form of a solution, powder, a micronised powder, sponge matrix, nano-fibre electrospun matrix, a hydrogel or in a lyophilised form.

"Sponge matrix" refers to a matrix which is soft, highly flexible, porous and hydrophilic in nature, made up of a blend of different polymers, synthetic and/or natural, with high flexibility and absorbent capacity. Such sponges can be used to absorb the extracts of the invention and release it at the surface of the human skin through contact.

"Nano-fibre electrospun matrix" refers to a matrix made of threads of polymer solutions or polymer melts up to fiber diameters in the order of some hundred nanometers. Such matrices are useful for medical purpose because they can be penetrated with cells or drug substances to treat or replace biological targets. For example, nanofibrous wound dressings have excellent capability to prevent microbial infections in wounds.

"Hydrogel" refers to a network of soluble collagen fibres that are prevented from dissociating by polymer entanglement and/or covalent cross-linking. Such polymer chains that are hydrophilic, resulting in a highly absorbent material. Hydrogels can also be formed from colloidal suspensions. The physical properties of hydrogels can be tuned depending on the route of manufacture.

"Lyophilised" indicates that the water has be removed from a substance by a step of frozen followed by a step where the substance is placed under a vacuum.

Preferably, the collagenous marine invertebrate extract is in the form of a solution, powder, a micronised powder, sponge matrix, nano-fibre electrospun matrix, a hydrogel, or in a lyophilised form.

In one embodiment, the collagenous marine invertebrate extract is administered in combination with at least one growth factor, preferably chosen among Platelet Rich Plasma (PRP), Epithelial Growth Factor (EGF), Transforming Growth Factor-Beta (TGF-B, TGF-B2, TGF-B3), Hepatocyte Growth Factor (HGF), Keratinocyte Growth Factor (KGF), Granulocyte-Monocyte Colony Stimulating Growth Factor, Platelet Derived Growth Factor, Insulin-like Growth Factor 1 (IGF1), basic Fibroblast Growth Factor (bFGF), and/or Vascular Endothelial Growth Factor (VEGF), and/or at least one antimicrobial compound, preferably selected from nano silver, penicillin, ofloxacin, tetracycline, aminoglycosides and erythromycin, flucloxacillin, clarithromycin, doxycycline, gentamicin, metronidazole, co-amoxiclav, co-trimoxazole (in penicillin), ceftriaxone, piperacillin with tazobactam, clindamycin, ciprofloxacin, vancomycin, teicoplanin, linezolid, and/or the standard of care antimicrobial agent, or any combination thereof.

In one embodiment, the collagenous marine invertebrate extract is administered at a dose from 0.01g/L to 200g/L per administration, preferably 1g/L to 20g/L per administration, more preferably 1 g/L to 20 g/L per administration. In other embodiments, the collagenous marine invertebrate extract is administered at a dose of 2g/L to 175g/L, 3g/L to 150g/L, 4g/L to 125g/L, 5g/L to 100g/L, 6g/L to 90g/L, 7g/L to 80g/L, 8g/L to 70g/L, 9g/L to 60g/L, 10g/L to 50g/L, 11g/L to 45g/L, 12g/L to 40g/L, 13g/L to 35g/L, 14g/L to 30g/L, 15g/L to 29g/L, 16g/L to 28g/L, 17g/L to 27g/L, 18g/L to 26g/L, or 19g/L to 25g/L.

In a second aspect, there is provided a pharmaceutical composition comprising the collagenous marine invertebrate extract according to the invention.

In one embodiment, the pharmaceutical composition further comprises at least one pharmaceutically acceptable compound. Preferably, said pharmaceutically acceptable compound is one or more selected from the group comprising: a growth factor, an antimicrobial, an antibiotic, an anti-inflammatory agent, and/or another Epidermolysis Bullosa treatment.

A "Growth factor" is a naturally occurring substance capable of stimulating cellular growth, proliferation, healing, and cellular differentiation.

An "Antimicrobial" is an agent that can kill or prevent the growth of microorganisms. Antimicrobial medicines can be grouped according to the microorganisms they act primarily against. For example, "antibiotics" are used against bacteria and antifungals are used against fungi.

"Anti-inflammatory agent" is a substance that has the property of reducing inflammation and/or swelling. Examples of anti-inflammatory agents include steroidal anti-inflammatory agents (e.g. corticosteroids) and non-steroidal anti-inflammatory drugs (i.e. NSAIDs).

"Other epidermolysis Bullosa treatment" refers to other compound in any type that would be efficient in the prevention or treatment of symptoms or effects of epidermolysis bullosa.

In some embodiments, the pharmaceutical compositions further comprise at least one growth factor. In preferred embodiments, the at least growth factor is Platelet Rich Plasma (PRP), Epithelial Growth Factor 38 (EGF), Transforming Growth Factor-Beta (TGF-B, TGF-B2, TGF-B3), Hepatocyte Growth Factor (HGF), Keratinocyte Growth Factor (KGF), Granulocyte-Monocyte Colony Stimulating Growth Factor, Platelet Derived Growth Factor, Insulin-like Growth Factor 1 (IGF1), basic Fibroblast Growth Factor (bFGF), and/or Vascular Endothelial Growth Factor (VEGF), or any combination thereof.

In some embodiments, the pharmaceutical compositions further comprise at least one antimicrobial. In preferred embodiments, the at least one antimicrobial is nano silver, penicillin, ofloxacin, tetracycline, aminoglycosides and erythromycin, flucloxacillin, clarithromycin, doxycycline, gentamicin, metronidazole, co-amoxiclav, co-trimoxazole (in penicillin), ceftriaxone, piperacillin with tazobactam, clindamycin, ciprofloxacin, vancomycin, teicoplanin, linezolid, and/or the standard of care antimicrobial agent., or any combination thereof.

In some embodiments, the pharmaceutical compositions further comprise at least one anti-inflammatory agent. In some embodiments, the at least one anti-inflammatory agent may be a steroidal anti-inflammatory agent such as a corticosteroid, or a non-steroidal anti-inflammatory drug (NSAID) such as aspirin salsalate, diflunisal, ibuprofen, ketoprofen, nabumetone, piroxicam, naproxen, diclofenac, indomethacin, or sulindac.

In one embodiment, said pharmaceutical composition is formulated in any form normally used for topical application, preferably a micronised powder, cream, ointment, mask, serum, milk, lotion, paste, foam, aerosol, stick, shampoo, conditioner, patch, hydroalcoholic or oily aqueous solution, an oil-in-water or water-in-oil or multiple emulsion, an aqueous or oily gel, a liquid, pasty or solid anhydrous product, an electrospun collagen nano-fibre matrix, a membrane and/or an oil dispersion in an aqueous phase using spherules, these spherules being polymeric nanoparticles such as nanospheres and nanocapsules or lipid vesicles of ionic and/or non-ionic type, more preferably an electrospun collagen nano-fibre matrix and/or a membrane.

In some embodiments, the pharmaceutical composition comprises at least one pharmaceutically acceptable excipient and/or carrier. Examples of suitable pharmaceutically acceptable excipients and carriers may include sterile water, olive oil, ethyl oleate, glycols, monosaccharides such as fructose, glucose and galactose; non-reducing disaccharides such as sucrose, lactose and trehalose; non-reducing oligosacchairdes such as raffinose and melezitose; non-reducing starch derived polysaccharide products such as maltodextrins, dextrans and cyclodextrins; and non-reducing alditols such as mannitol and xylitol. Further suitable excipients include Polyethylene glycol (PEG), cellulose preparations such as maize starch, wheat starch, rice starch, potato starch, gelatin, gum tragacanth, and/or polyvinylpyrrolidone.

In a third aspect, there is provided a method for manufacturing a collagenous marine invertebrate extract, comprising the steps of:
(a) heating marine invertebrate soluble collagen between 40°C - 100°C for between 10 minutes to 120 minutes, preferably between 60°C - 80°C for 45 minutes to 90 minutes, so as to hydrolyse the material;
(b) recovering the hydrolysed material.

In some embodiments of the method for manufacturing a collagenous marine invertebrate extract, step (a) comprises the addition of a protease enzyme.

Protease, or peptidase are enzymes which catalyse the hydrolysis of peptide bonds present in proteins and polypeptides. Proteases are divided into two groups: exopeptidases and endopeptidases, based on the site of action on polypeptide chains. The exopeptidases act on the ends of polypeptide chains and endopeptidases act randomly in the inner regions of polypeptide chains (Raveendran et al. 2018. Food Technol Biotechnol., 56(1): 16-30.). Preferably, proteases according to the invention are capable of hydrolysing collagen, for example pepsin, matrix metallopeptidase, papain, and/or procollagen peptidase. More preferably, said protease is pepsin or a pepsin homologue.

The method for manufacturing a collagenous marine invertebrate extract may further comprise purification of the material obtained after step (a) prior to step (b). In a preferred embodiment, the material is purified by membrane filtration or chromatographic purification.

There are multiple methods for 'isolating', or 'purifying' collagenous material from the anatomical milieu of marine invertebrates and such methods are well known to the skilled person. For example, collagenous material can be purified from jellyfish by acid extraction, whereby different anatomical parts of the jellyfish are bathed in an acidic solution. 'Bathing', or 'bathed', refers to the process of incubating the jellyfish in the acid solution for a sufficient amount of time in order to liberate the collagenous material. An alternative method for purification of collagenous material is enzyme extraction, whereby the jellyfish is incubated with at least one proteolytic enzyme for a sufficient amount of time and under conditions that favour the degradation of the anatomical milieu in order to liberate the collagenous material. The exact temperature, pH and incubation time of the enzyme extraction method will vary depending on the proteolytic enzyme used. The most suitable conditions will be well known to the skilled person. By way of non-limiting example, the enzyme pepsin can be incubated with jellyfish material under acidic conditions in order to liberate the collagenous material.

### DESCRIPTION OF THE FIGURES

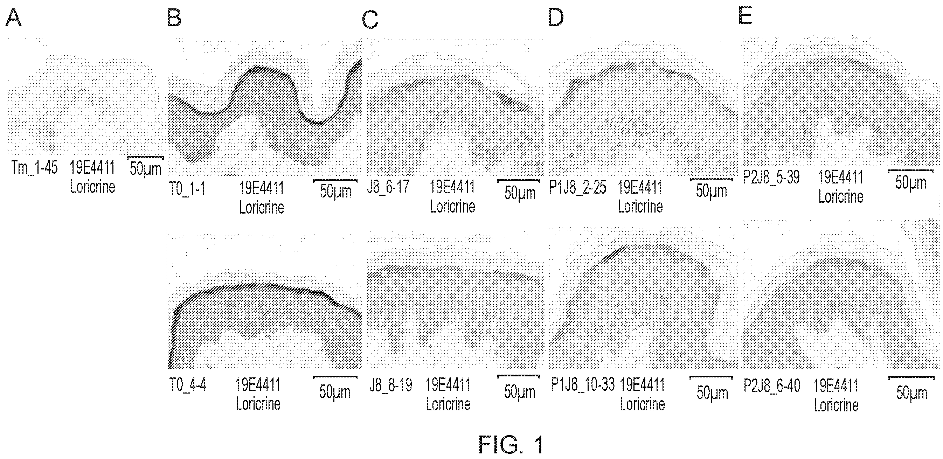

**Figure 1** shows the results of the immunolabeling of Loricrin. **(A)** shows the result of the negative control without the primary antibody; **(B)** shows the control batch at Day 0 (J0); **(C)** shows the control batch at Day 8 (J8); **(D)** shows the P1 batch at day 8 (J8); **(E)** shows the P2 batch at day 8 (J8).

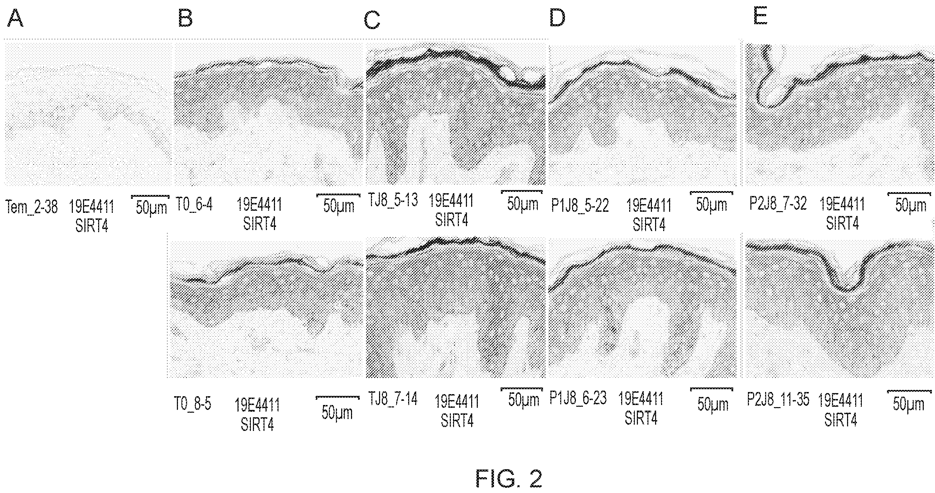

**Figure 2** shows the results of the immunolabeling of SIRT-4. **(A)** shows the result of the negative control without the primary antibody; **(B)** shows the control batch at Day 0 (30); **(C)** shows the control batch at Day 8 (J8); **(D)** shows the P1 batch at day 8 (J8); **(E)** shows the P2 batch at day 8 (J8).

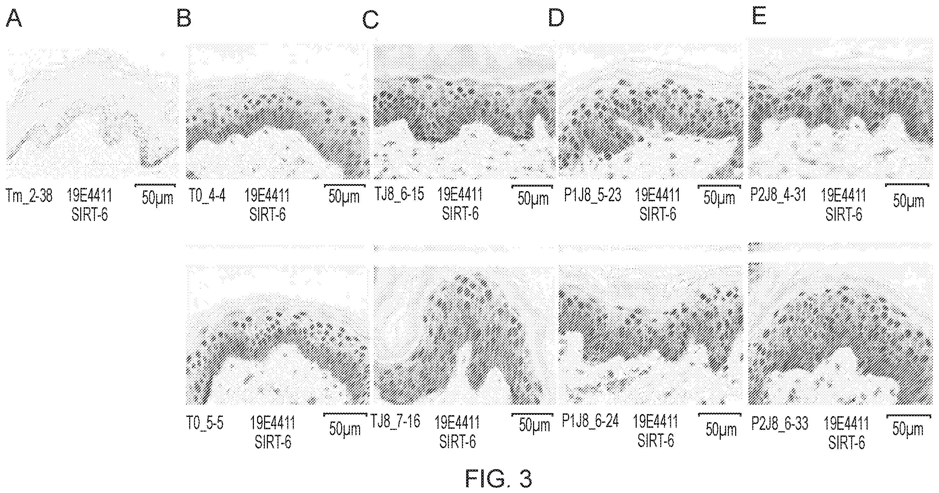

**Figure 3** shows the results of the immunolabeling of SIRT-6. **(A)** shows the result of the negative control without the primary antibody; **(B)** shows the control batch at Day 0 (J0); **(C)** shows the control batch at Day 8 (J8); **(D)** shows the P1 batch at day 8 (J8); **(E)** shows the P2 batch at day 8 (J8).

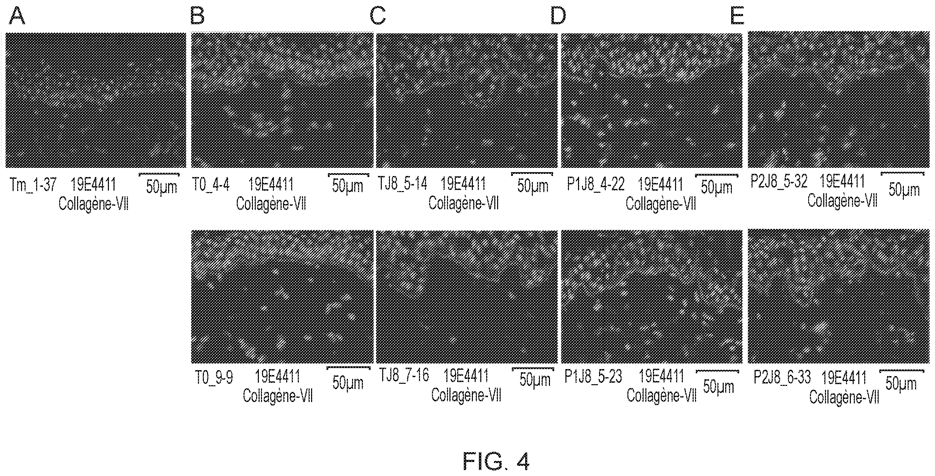

**Figure 4** shows the results of the immunolabeling of collagen VII. **(A)** shows the result of the negative control without the primary antibody; **(B)** shows the control batch at Day 0 (J0); **(C)** shows the control batch at Day 8 (J8); **(D)** shows the P1 batch at day 8 (J8); **(E)** shows the P2 batch at day 8 (J8).

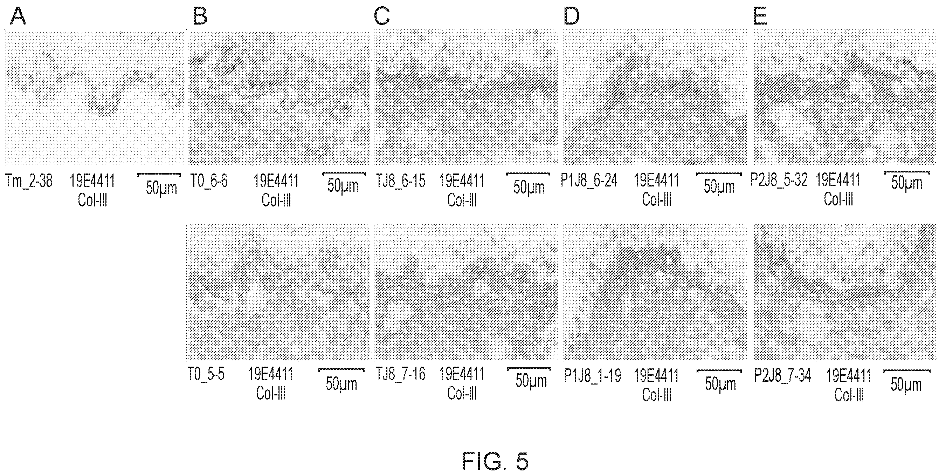

**Figure 5** shows the results of the immunolabeling of collagen III. **(A)** shows the result of the negative control without the primary antibody; **(B)** shows the control batch at Day 0 (J0); **(C)** shows the control batch at Day 8 (J8); **(D)** shows the P1 batch at day 8 (J8); **(E)** shows the P2 batch at day 8 (J8).

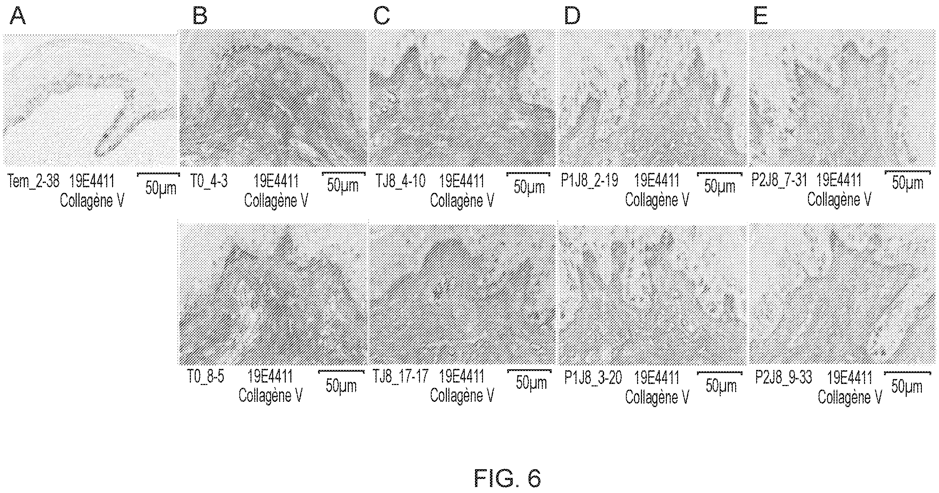

**Figure 6** shows the results of the immunolabeling of collagen V. **(A)** shows the result of the negative control without the primary antibody; **(B)** shows the control batch at Day 0 (J0); **(C)** shows the control batch at Day 8 (J8); **(D)** shows the P1 batch at day 8 (J8); **(E)** shows the P2 batch at day 8 (J8).

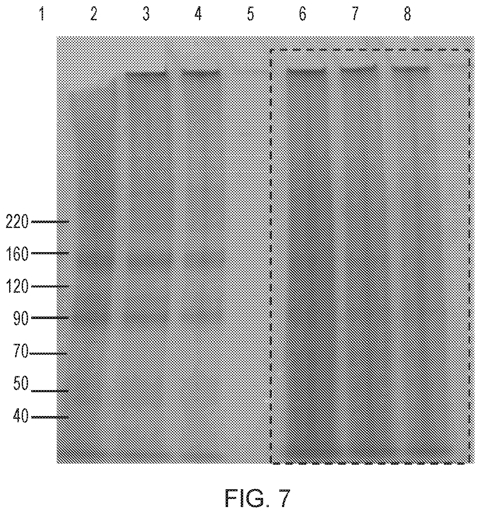

**Figure 7** shows a representative SDS PAGE image (3-8% Tris acetate gel) demonstrating the effect of heat-treatment on the size distribution of proteins in collagenous jellyfish extracts. Lane 1: molecular weight marker; Lanes 2-4: native collagenous jellyfish extracts; Lane 5: empty; Lanes 6-8: heat-treated collagenous jellyfish extracts.

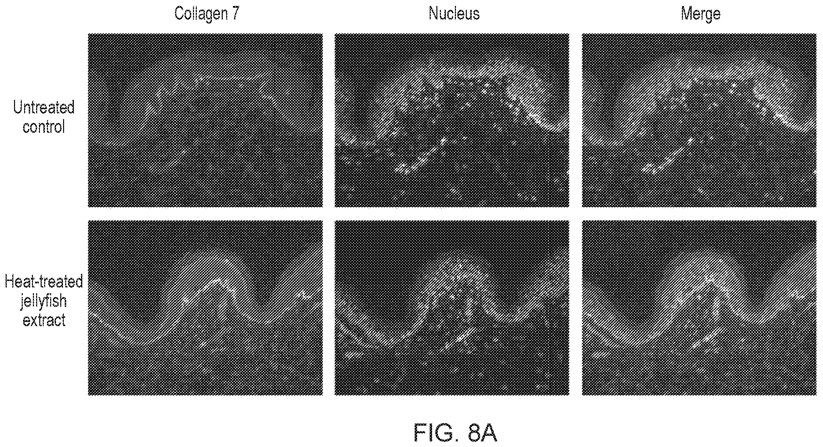

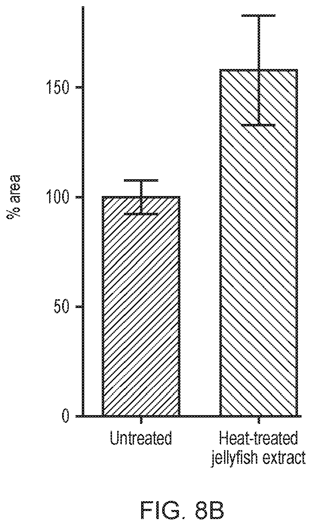

**Figure 8** shows the results of treating healthy human skin explants with heat-treated collagenous jellyfish extract. **(A)** Representative immunofluorescence image of explant slices stained for collagen 7 with and without heat-treated collagenous jellyfish extract treatment. **(B)** Graph showing the average percentage area of collagen 7 staining in untreated and treated explant slices (n = 10). The percentage area of collagen 7 staining is normalised to the untreated control (100%) and the error bars represent standard error of the mean.

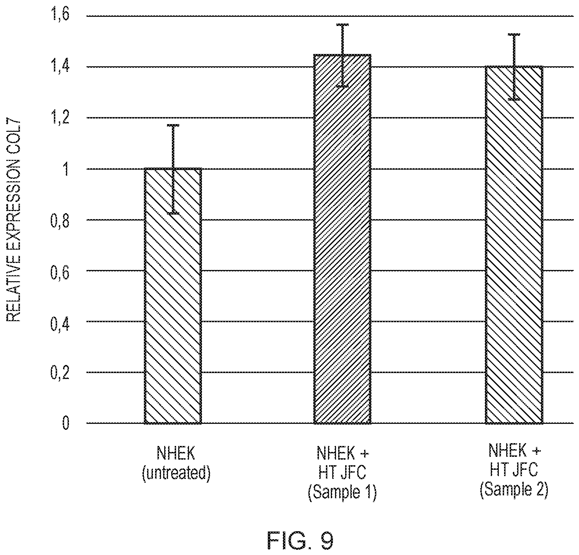

**Figure 9** shows COL7 gene expression in healthy human epidermal keratinocytes (NHEKs) following treatment with heat-treated collagenous jellyfish extract, as determined by semi-quantitative RT-PCR (Applied Biosystems 7300 Real Time PCR System). The average expression level of COL7 mRNA in treated NHEKs (n = 3) is shown relative to untreated control cells (1.0) and the error bars represent standard error of the mean.

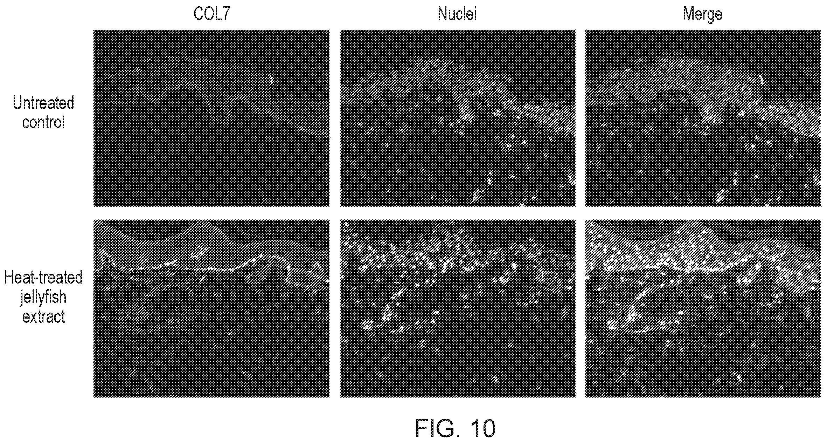

**Figure 10** shows a representative image immunofluorescence image of explant slices stained for collagen 7 with and without heat-treated collagenous jellyfish extract treatment in accordance with the analysis described in Example 10. Analogous images were generated for explant slices stained for laminin 5 and keratin 5.

### EXAMPLES

### Example 1: Characterisation of R. Pulmo collagen

The physicochemical characteristics of *R. pulmo* collagens (native or hydrolysed by heat-treatment) are shown in Table 1.

### Table 1: Physicochemical characteristics of R. pulmo collagens

| **characteristics** | **Collagen** | **Collagenous extract hydrolysate** |
|---|---|---|
| Aspect | Liquid | Liquid |
| Colour | Colourless to pale yellow | Colourless to pale yellow |
| Solvent | Acetic acid 0,1 M | Acetic acid 0,5 M |
| Concentration | 3-6 mg.mL⁻¹ | 3-10 mg.mL⁻¹ (8,25 mg.mL⁻¹) |
| Turbidity | Clear to slightly cloudy | Clear to slightly cloudy |
| Protein Level | > 80% | > 80% |
| pH | 2,8-3,2 | 2,8-3,5 |

Jellyfish collagen was also tested for cytotoxicity. The biocompatibility of jellyfish collagen (3.7 mg.mL-1) was evaluated by a measurement of cytotoxicity on a cell culture (murine fibroblasts L929), according to the ISO 10993-5 2009 method by Contact Indirect. The results indicate that neither the native nor the hydrolysed collagenous jellyfish extract resulted in detectable cytotoxicity under the test conditions used.

### Example 2: Evaluation of the Jellyfish collagenous hydrolysate by immunolabelling of the synthesis of loricrin, sirtuin-4 and 6, and collagen III, V and VII

Twelve explants of about 1 cm in diameter were prepared from an abdominoplasty surgery of a 51-year-old woman with dark skin. The explants were placed in a BEM (BIO-EC's Explants Medium) environment at 37°C in a humid atmosphere, enriched with 5% CO2. Table 2 below shows the treatment conditions used on the obtained explants.

**Table 2: Treatment conditions of explants**

| **Lots** | **Names** | **Concentration** | **N° explants** | **End** |
|---|---|---|---|---|
| T0 | Abdominoplasty control | / | 3 | J0 |
| T | Untreated control | / | 3 | J8 |
| P1 | Product 1 | 0.1 mg/mL | 3 | J8 |
| P2 | Product 2 | 1 mg/mL | 3 | J8 |

P1 and P2 are *R. Pulmo* collagen hydrolysates at different concentrations (as shown in Table 3 below). J0 and J8 mean Day 0 and 8 respectively.

**Table 3: Concentrations and names of the products tested:**

| Product | Concentrations |
|---|---|
| P1 | 0.1 mg/mL |
| P2 | 1 mg/mL |

The purpose of this study was to carry out the following immunolabelling on mounted slices from the treated explants:
- Immunolabelling of loricrin;
- Immunolabeling of sirtuin-4;
- Immunolabeling of sirtuin-6;
- Immunolabelling of collagen VII;
- Immunolabelling of collagen III;
- Immunolabelling of collagen V.

The products P1 (0.1 mg/mL) and P2 (1 mg/mL) were applied to the explants described above, and compared after 8 days with an abdominoplasty control (T0) and a non-treated control (T).

### Histological treatments

Cuts of 5 µm were made using a microtome type Minot, Leica RM2125 and mounted on Superfrost^{®} histological glass slides. Cuts of 7 µm were made using a Leica CM 3050 cryostat and mounted on Superfrost^{®} Plus silanised histological glass slides.

Microscopic observations were made by optical microscopy, using a Leica microscope such as DMLB or Olympus BX43. The shots were taken with an Olympus DP72 camera and CellD software.

### a. Immunolabelling of loricrin

Loricrin was labelled on formalinised paraffin sections with a polyclonal antibody against loricrin (Covance, ref. PRB-14P), diluted to 1/1600th in PBS-BSA 0.3%-Tween 20 to 0.05% for 1 h at room temperature, with a biotin amplifier/streptavidin system, revealed in VIP, a violet peroxidase substrate (Vector, ref. SK 4600). The labelling was carried out using an immunolabelling automaton (Autostainer, Dako) and was evaluated by microscopic examination. Lots concerned: T0, TJ8, P1J8 and P2J8.

### b. Immunolabeling of sirtuin-4 (SIRT-4)

Sirtuin-4 was labelled on formalin paraffin sections with a polyclonal antibody against sirtuin-4 (GeneTex, ref. GTX51798), diluted 1/100th in PBS-BSA 0.3%-Tween 20 to 0.05% for 1 h at room temperature, with a biotin amplifier/streptavidin system, revealed in VIP, a violet peroxidase substrate (Vector, ref. SK 4600). The labelling was carried out using an immunolabelling automaton (Autostainer, Dako) and was evaluated by microscopic examination. Lots concerned: T0, TJ8, P1J8 and P2J8.

### c. Immunolabeling of sirtuin-6 (SIRT-6)

Sirtuin-6 was labelled on formalin paraffin sections with a polyclonal antibody against sirtuin-6 (Novus Biologicals, ref. NB100-2522), diluted to 1/100th in PBS-BSA 0.3%-Tween 20 to 0.05% for 1 h at room temperature, with a biotin/streptavidin amplifier system, revealed in VIP, a purple peroxidase substrate (Vector, ref. SK 4600). The labelling was carried out using an immunolabelling automaton (Autostainer, Dako) and was evaluated by microscopic examination. Lots concerned: T0, TJ8, P1J8 and P2J8.

### d. Immunolabelling of collagen VII

Collagen VII was labelled on frozen sections with a monoclonal antibody against collagen VII (Santa Cruz, ref. sc-53226, clone LH 7.2), diluted 1/100th in PBS-BSA 0.3%-Tween 20 to 0.05% for 1 h at room temperature, and revealed in AlexaFluor 488 (Lifetechnologies, ref. A11008). The labelling was carried out using an immunolabelling automaton (Autostainer, Dako) and was evaluated by microscopic examination. Lots concerned: T0, TJ8, P1J8 and P2J8.

### e. Immunolabelling of collagen III

Collagen III was labelled on frozen sections with a polyclonal antibody against collagen III (SBA, ref. 1330-01), diluted to 1/100th in PBS-BSA 0.3%-Tween 20 to 0.05% for 1 h at room temperature, with a biotin/streptavidin amplifier system, revealed in VIP, a purple peroxidase substrate (Vector, ref. SK 4600). The marking was done manually and evaluated by microscopic examination. Lots concerned: T0, TJ8, P1J8 and P2J8.

### f. Immunolabelling of collagen V

Collagen V was labelled on formalin paraffin sections with a polyclonal antibody against collagen V (Novotec, ref. 20511), diluted to 1/100th in PBS-BSA 0.3%-Tween 20 to 0.05% for 1 h at room temperature, with a biotin/streptavidin amplifier system, revealed in VIP, a violet peroxidase substrate (Vector, ref. SK 4600). The labelling was carried out using an immunolabelling automaton (Autostainer, Dako) and was evaluated by microscopic examination. Lots concerned: T0, TJ8, P1J8 and P2J8.

### Results and Conclusions

The results for **Loricrin** are shown in Figure 1. At Day 0, on the control batch T0, the loricrin marking is fairly clear to clear in the granular layer. At Day 8, on the control batch TJ8, loricrin expression is moderate in the granular layer. Relative to control lot TJ8, both P1 and P2 induce a slight increase in loricrin expression.

The results for **SIRT-4** are shown in Figure 2. At Day 0, on the control batch T0, the marking of sirtuin-4 is fairly clear in the epidermis. At Day 8, on the control lot TJ8, the expression of sirtuin-4 is quite clear to clear in the epidermis. Relative to control lot TJ8, P1 induces a slight or moderate decrease in sirtuin-4 expression and P2 induces a moderate decrease in sirtuin-4 expression.

The results for **SIRT-6** are shown in Figure 3. At Day 0, on the control batch T0, the marking of sirtuin-6 is fairly clear to clear in the epidermis. At Day 8, on the control lot TJ8, the expression of sirtuin-6 is clear in the epidermis. Relative to control lot TJ8, P1 induces a slight decrease in sirtuin-6 expression and P2 does not induce any observable change in expression of sirtuin-6.

The results for **Collagen VII** are shown in Figure 4. At Day 0, on the control batch T0, the labelling of collagen VII is weak along the dermal-epidermal junction. At Day 8, on the control lot TJ8, collagen VII expression is low to moderate along the dermo-epidermal junction. Relative to control lot TJ8, both P1 and P2 induce a moderate increase in Collagen VII expression.

The results for **Collagen III** are shown in Figure 5. At Day 0, on the control batch T0, the marking of collagen III is quite clear in the dermis papillary. At day 8, on the control lot TJ8, collagen III expression is clearly low in the papillary dermis. Relative to control lot TJ8, P1 and P2 did not induce an observable change in expression of Collagen III.

The results for **Collagen V** are shown in Figure 6. At day 0, on the control batch T0, the marking of collagen V is clear to very clear in the papillary dermis. At day 8, on the control batch TJ8, the expression of collagen V is clear in the papillary dermis. Relative to control lot TJ8, P1 induces a moderate decrease in Collagen V expression and P2 induces a very clear decrease in Collagen V expression.

A summary of the results obtained in the above-described immunolabelling experiments is shown in Table 4.

**Table 4: Immunolabelling results for P1 and P2 products**

| *Variation vs TJ8* | **P1** | **P2** |
|---|---|---|
| **Loricrin** | Slight Increase | Slight decrease |
| **Sirtuin-4** | Slight decrease | Moderate decrease |
| **Sirtuin-6** | Slight decrease | No Change |
| **Collagen VII** | Moderate Increase | Moderate Increase |
| **Collagen III** | No Change | No Change |
| **Collagen V** | Moderate decrease | Very clear decrease |

| | | |
|---|---|---|
| Scale = High Decrease < Very clear < clear < Fairly clear < Moderate < Slight Decrease < No Change < Slight Increase < Moderate < Fairly clear < clear < Very clear < Strong Increase | | |

### Example 3: In vitro study of the effect of Rhizostoma pulmo collagenous extract on a primary culture of human fibroblast

After application of hydrolysed collagenous extracts according to the invention to human fibroblast cells for 24 hours, intracellular and extracellular collagen was quantified with the red dye Sirius (Direct red 80), which has a particular affinity for the triple helix structure (Gly-X-Y)n of native collagen (collagen type I to V). The absorbance of the dye and collagen complex was measured at 540 nm. The total protein fraction was evaluated, after sonication, using the Bradford method (Bradford et al. Anal Biochem 1976; 72:248-54).

### Test system

Primary human fibroblasts were prepared and the cells were confirmed as being free of mycoplasma.

The cells were cultured in DMEM medium 4.5 g/L glucose, 2 mM stabilised L-glutamine or glutamine, 10% heat inactivated foetal calf serum (FSC), 50 IU/ml penicillin, 50 µg/ml streptomycin. The cells were kept in a humid atmosphere (37°C, 5% CO2). The cells were used at passage 6.

### Reference elements

*Negative control*: culture medium with 1% SVF.
*Positive control*: Transforming growth factor β1 (TGFβ) at 10 ng/ml in culture medium with 1% SVF

### Definition of the series

Five concentrations of collagenous extracts according to the invention were tested. The initial concentration of hydrolysed collagenous extract (8.25 mg/ml) was reduced to 500, 100, 10, 10, 5 and 1 µg/ml. Each condition and reference element was tested in triplicate.

### Test protocol:

*Cell seeding*: The cells were seeded at 50,000 cells/cm² in culture plates of 24 wells and then incubated overnight (37°C, 5% CO2).

*Contact between the cells and the test item or reference items*: Dilutions of the test items or reference items were performed in culture medium at 1% SVF. The culture medium was aspirated and replaced by 500 µl of the different concentrations of the test items or reference items. The plates were incubated for 24 hours ± 1 hour (37° C, 5% CO2).

*Assessment of collagen synthesis and cell density*: The culture medium of each well was then sampled and the cell layer was rinsed with 500 µl of concentrated protease inhibitor cocktail 2 times. The entire set, culture medium and inhibitor, was pooled in the same tube.

The cell layer of each well was scraped off in 500 µl of concentrated protease inhibitor cocktail 1 time and the wells were rinsed again with 500 µl of cocktail 1 time. The two volumes, which constitute the extracellular matrix, were combined in a single tube and treated with an ultrasound probe for 40 seconds.

The collagen of each fraction was complexed with the red dye Sirius and, after centrifugation and washing with 0.1M HCl, the pellet was solubilised in 0.5M NaOH. The absorbance of the dye and collagen complex was measured at 540 nm. A calibration range has been established under the same conditions between 0 and 10 µg per collagen tube.

The amount of total protein was evaluated using the Bradford method (Bradford et al. Anal Biochem 1976; 72:248-54) against a calibration range established from a BSA solution at 400 µg/ml in PBS. 30 µl of each sample was mixed with 280 µl of Bradford reagent in a 96-well plate. The plate was incubated for about 15 minutes at room temperature in the dark. Absorbance was measured at 620 nm against a reagent blank consisting of the Bradford reagent.

### Results

The results of the above-described experiments are summarised in Table 5 below.

**Table 5: Stimulation of collagen synthesis by the Rhizostoma pulmo collagenous extract.**

| | Protein Concentrati on | Collagen cell matrix | | | Collagen culture medium | | |
|---|---|---|---|---|---|---|---|
| Concentrati on | µg protein / wells / | Averag e Standa rd deviati on | µg collage n / well/ | % Stimulati on / Negative control | Averag e Standa rd deviati on | µg collage n / well/ | % Stimulati on / Negative control |
| *Rhizostoma pulmo* collagen hydrolysate | | | | | | | |
| 500 µg/ml | 54.78 | 0.256 | 2.60 | 73 | 0.233 | 2.37 | 0 |
| | 3.8 | 0.015 | | | 0.023 | | |
| 100 µg/ml | 64.64 | 0.251 | 2.55 | 70 | 0.250 | 2.54 | 7 |
| | 9.0 | 0.042 | | | 0.040 | | |
| 10 µg/ml | 62.17 | 0.272 | 2.77 | 84 | 0.252 | 2.56 | 0 |
| | 3.5 | 0.022 | | | 0.050 | | |
| 5 µg/ml | 56.81 | 0.205 | 2.09 | 39 | 0.226 | 2.30 | 0 |
| | 3.9 | 0.020 | | | 0.028 | | |
| 1 µg/ml | 50.58 | 0.195 | 1.99 | 32 | 0.225 | 2.29 | 0 |
| | 7.0 | 0.043 | | | 0.032 | | |
| Positive control | 65.87 | 0.210 | 2.14 | 42 | 0.217 | 2.20 | 0 |
| TGF-β 10ng/ml | 2.8 | 0.031 | | | 0.029 | | |
| Negative control | 50.14 | 0.148 | 1.50 | | | | |
| | 4.1 | 0.029 | | | | | |

Treatment with the hydrolysed collagenous jellyfish extract resulted in a significant increase in collagen synthesis in the cellular matrix with a maximum increase of 84% when 10 µg/ml of that collagenous product was used.

### Example 4: Comparative data against hydrolysed fish collagen (Peptan SR Marine)

The same experiments as those performed in Example 3 were performed using hydrolysed fish collagen (Peptan SR Marine) at 500, 100, and 10 µg/ml. The results obtained when using hydrolysed jellyfish and fish collagen are compared in Table 6 below.

**Table 6: Stimulation of collagen synthesis by the hydrolysed jellyfish collagenous extract and hydrolysed fish collagen.**

| | Protein Concentrati on | Collagen cell matrix | | | Collagen culture medium | | |
|---|---|---|---|---|---|---|---|
| Concentrati on | µg protein / wells / | Averag e Standar d deviatio n | µg collage n / well/ | Concentrati on | µg protei n / wells / | Averag e Standar d deviatio n | µg collage n / well/ |
| Rhizostoma pulmo collagen hydrolysate | | | | | | | |
| 500 µg/ml | 54,78 | 0,256 | 2,60 | 73 | 0,233 | 2,37 | 0 |
| | 3,8 | 0,015 | | | 0,023 | | |
| 100 µg/ml | 64,64 | 0,251 | 2,55 | 70 | 0,250 | 2,54 | 7 |
| | 9, | 0,042 | | | 0,040 | | |
| 10 µg/ml | 62,17 | 0,272 | 2,77 | 84 | 0,252 | 2,56 | 0 |
| | 3,5 | 0,022 | | | 0,050 | | |
| 5 µg/ml | 56,81 | 0,205 | 2,09 | 39 | 0,226 | 2,30 | 0 |
| | 3,9 | 0,020 | | | 0,028 | | |
| 1 µg/ml | 50,58 | 0,195 | 1,99 | 32 | 0,225 | 2,29 | 0 |
| | 7,0 | 0,043 | | | 0,032 | | |
| Hydrolysed fish collagen | | | | | | | |
| 500 µg/ml | 95,22 | 0,144 | 1,47 | 0 | 0,254 | 2,58 | 9 |
| | 1,2 | 0,019 | | | 0,021 | | |
| 100 µg/ml | 66,09 | 0,214 | 2,18 | 45 | 0,212 | 2,15 | 0 |
| | 0,6 | 0,030 | | | 0,015 | | |
| 10 µg/ml | 50,87 | 0,249 | 2,53 | 68 | 0,222 | 2,26 | 0 |
| | 6,1 | 0,012 | | | 0,031 | | |
| Positive control | 65,87 | 0,210 | 2,14 | 42 | 0,217 | 2,20 | 0 |
| TGF-β 10ng/ml | 2,8 | 0,031 | | | 0,029 | | |
| Negative control | 50,14 | 0,148 | 1,50 | | | | |
| | 4,1 | 0,029 | | | | | |

Hydrolysed fish collagen has a maximum effect of 68% at a concentration of 10 µg/ml. The response appears to be inversely proportional to the concentration, and remains lower than the values obtained with hydrolysed jellyfish collagenous extract.

### Example 5: Evaluation of hydrolysed collagenous extracts on gene expression in human skin explants

### Biological Model

NativeSkin^{®} skin explant has been used, which is a biopsy of human normal skin embedded in a solid and nourishing matrix. The epidermal surface is maintained in contact with air to allow topical application.

The explants used were prepared from a 32-year-old woman's abdominoplasty, corresponding to a pigmentation of 3 on the Fitzpatrick scale.

The hydrolysed collagenous extract used in this example was concentrated at 100 µg/ml (0.1 g/L).

The experiment was carried out in triplicate in the following conditions:
- Skin explant (control) untreated (n=3)
- Skin explant + Hydrolysed jellyfish collagenous extract (BT-10R&D) 0,1g/L (n=3)
- Skin explant + Hydrolysed fish collagen (2132415-1/28052018) 0,1g/L (n=3)

The explants were treated with the products for 2 days with a new application of the product in the morning and in the evening.

NB: Hydrolysed fish collagen seems to weaken the epidermis. Indeed, after treatment the explants were prepared for RNA extraction by punching them to remove skin unexposed to the product. It was observed that the epidermis was slightly detached from the dermis.

At the end of the culture phase, the explants were removed from their inserts and microfluidic RT-qPCR was used to characterise the gene expression of skin explants.

### Results/Conclusions

The specificity of each primer pair was verified for all conditions by dissociation curve analysis and the problematic points with double peaks are removed from the analysis. Each CT was then normalised using the reference genes and the control condition in order to calculate the ΔΔCT and the relative expression was calculated as a function of ΔΔCT using the formula 2-ΔΔCT.

For each condition, the standard error to the mean (SEM) was calculated and the points with too large an SEM value (> 40% difference between the SEM and the average of the 3 values) were removed from the analysis. The results are expressed as a percentage of overexpression or subexpression relative to the reference condition.

Changes in the expression of the genes of interest induced by the treatments are shown in Table 7 (heat-treated collagenous jellyfish extract) and Table 8 (hydrolysed fish collagen peptides) below.

**Table 7: Effect of the R. Pulmo hydrolysed collagenous extract on the gene expression of human skin explants.**

| Genes | % increase or decrease in expression |
|---|---|
| COL3A1 | +60% |
| COL5A1 | +110% |
| DCN | +140% |
| FBN1 | +80% |
| FBN2 | +490% |
| LOX | +80% |
| LUM | +270% |
| SPARC | +100% |
| LOXL1 | -90% |
| CDC42 | +60% |
| COL7A1 | +170% |
| LAMA5 | +70% |
| PI3 | -90% |
| EGF | +130% |
| SIRT4 | +340% |
| SIRT6 | +60% |
| HBEGF | -60% |
| MGST1 | +230% |
| TNFA | +70% |
| EPPK1 | +100% |
| EVPL | +80% |
| FLG | +60% |
| LOR | +130% |
| MMP3 | -80% |
| POMC | +140% |
| DKK1 | +230% |

Hydrolysed *R. Pulmo* collagenous extract was able to increase the synthesis of COL3A1, COL5A1, DCN, FBN1, FBN2, LOX, LUM, SPARC, CDC42, COL7A1, LAMA5, PI3, EGF, SIRT4, SIRT6, MGST1, TNFA, EPPK1, EVPL, FLG, LOR, POMC, DKK1, and decrease the synthesis of MMP3, HBEGF, PI3, and LOXL1.

**Table 8: Effect of the fish hydrolysed collagen according to the invention on the gene expression of human skin explants.**

| Genes | % increase or decrease in expression |
|---|---|
| LOXL1 | +180% |
| P4HA1 | +120% |
| COL1A1 | - 90% |
| COL5A1 | -60% |
| DCN | -70% |
| SPARC | -70% |
| CD44 | +60% |
| CDC42 | +80% |
| COL4A1 | +120% |
| CTNNA1 | +90% |
| ITGA2 | +170% |
| LAMC2 | +430% |
| PXN | +200% |
| SDC4 | +440% |
| COL17A1 | -70% |
| COL7A1 | -90% |
| FN1 | -60% |
| LAMA5 | -90% |
| SDC1 | -60% |
| MMP9 | -80% |
| PI3 | +3230% |
| TIMP1 | +130% |
| MMP1 | +2390% |
| MMP3 | +180% |
| AQP1 | -60% |
| AQP3 | -60% |
| HBEGF | +1000% |
| SIRT6 | +140% |
| TINF2 | +70% |
| IGFBP6 | +140% |
| GLRX | +70% |
| SIRT3 | +80% |
| SOD2 | +570% |
| CAT | -70% |
| MSRB2 | -90% |
| COX2 | +240% |
| IL1A | +4350% |
| IL6 | +380% |
| IL8 | +9280% |
| S100A7 | +790% |
| TNFA | +1150% |
| IVL | +540% |
| TGM1 | +520% |
| EVPL | -60% |
| FLG | -90% |
| LOR | -60% |
| POMC | +90% |
| DKK1 | +200% |

Hydrolysed fish collagen is able to increase the synthesis of LOXL1, P4HA1, CD44, CDC42, COL4A1, CTNNA1, ITGA2, LAMC2, PXN, SDC4, PI3, TIMP1, MMP1, MMP3, HBEGF, SIRT6, TINF2, IGFBP6, GLRX, SIRT3, SOD2, COX2,IL1A,IL6,IL8, S100A7, TNFA, IVL, TGM1, POMC, DKK1, and decrease the synthesis of COL1A1, COL5A1, DCN, SPARC, COL17A1, COL7A1, FN1, LAMA5, SDC1, MMP9, AQP1, AQP3, CAT, MSRB2, EVPL, FLG, LOR.

These results indicate that the *R. Pulmo* collagenous extract is capable of effectively activating expression of the genes COL3A1, FBN1, FBN2, SPARC, LUM, COL7A1, LAMA5, CDC42, EGF, SIRT4, SIRT6, EPPK, LOR, and TNFA and also decreasing expression of MMP3
As noted above, the *R. Pulmo* collagenous extract increases the expression of COL7A1 by +170%. A study by Izmiryan et al (Ex vivo COL7A1 correction for recessive dystrophic epidermolysis bullosa using CRISPR/Cas9 and homology-directed repair (2018). Molecular Therapy-Nucleic Acids, 12, 554-567.), indicated that re-expression of COL7A1 transcripts estimated to be up to 15% in RDEB cells and up to 19% in skin grafts was sufficient to allow anchoring fibril formation with no dermal-epidermal separation. Accordingly, the effects seen with the *R.Pulmo* collagenous extract represent a promising opportunity for treating EB.. That increase in the synthesis of COL7A1 suggests that the *R.Pulmo* collagenous extract has therapeutic relevance in the context of EB treatment.

In contrast, the fish collagen hydrolysate decreases the synthesis of many EB relevant genes, including COL7A1.

### Example 6: A study to determine the effect of heat treated jellyfish collagenous extract on the activation of genes ex-vivo associated with EB in human skin biopsies

### Methodology

Collagenous jellyfish extracts and control samples were tested *ex vivo* on healthy skin explants NativeSkin^{®}, a full-thickness skin biopsy embedded in a solid and nourishing matrix while its epidermal surface is left in contact with air.

Hydrolysis of collagenous extracts was carried out by heating the extract to 80°C for 60 minutes. Figure 7 shows a representative SDS-PAGE demonstrating the effect of heat-treatment on the size distribution of the proteins present in the collagenous jellyfish extracts.

Explants were tested under the following conditions:
- No extract treatment;
- Exposure to untreated collagenous jellyfish extract (0.1mg/mL);
- Exposure to heat-treated collagenous jellyfish extract (0.1mg/mL);
- Exposure to heat-treated collagenous mammalian extract (0.1mg/mL).

The explants were exposed to the test extracts for 24 hours. After that incubation, RNA from the skin explants was extracted and then reverse-transcribed into cDNA. The expression of 90 genes was assessed in triplicate by microfluidic technology RT-qPCR (Biomark^{®} - Fluidigm) and normalised to the expression of reference genes. In order to confirm an activating or inhibitory effect, values were compared to the untreated control. The results were expressed in terms of a expression rate relative to the untreated control.

### Results

Significant changes in EB-associated gene expression in human explants following different treatments are summarised in Table 9 below (as measured by microfluidic RT-qPCR).

**Table 9: Summary of significantly changed expression of EB-associated genes under in differently treated human explants**

| **Explant** | **EB-associated gene** | **Increase in expression relative to untreated control** |
|---|---|---|
| Untreated collagenous jellyfish extract 1O (negative control) | ITGA6 | +150% |
| Heat-treated collagenous jellyfish extract 1O | COL7A | +60% |
| | DSP | +160% |
| | ITGA6 | +460% |
| | ITGB4 | +260% |
| | KRT5 | +240% |
| | LAMB3 | +240% |
| | LAMC2 | +170% |
| Heat-treated collagenous jellyfish extract 2U | COL7A | +80% |
| | DSP | +70% |
| | ITGA6 | +200% |
| | ITGB4 | +50% |
| | KRT5 | +50% |
| Heat-treated collagenous jellyfish extract 3WU | DSP | +50% |
| | ITGB4 | +100% |
| | KRT5 | +70% |
| | LAMB3 | +70% |
| | LAMC2 | +110% |
| Heat-treated collagenous jellyfish extract 1OF | COL7A | +100% |
| | DSP | +50% |
| | ITGA6 | +180% |
| Heat-treated collagenous jellyfish extract 1O2H | ITGA6 | +240% |
| | ITGB4 | +60% |
| | LAMB3 | +80% |
| | LAMC2 | +70% |
| Heat-treated collagenous mammalian extract 1OF | ITGA6 | +60% |

The results in Table 9 illustrate that increased expression of key EB-associated genes is closely associated with the treatment of explants with heat-treated collagenous jellyfish extracts and was generally absent in mammalian extracts treated in the same way. Non-hydrolysed collagenous jellyfish extracts did not activate EB-associated gene expression in the same as heat-treated extracts, strongly suggesting that the observed effects are specific to hydrolysed collagenous jellyfish extracts.

In some explants exposed to heat-treated collagenous jellyfish extract increases in the expression of other EB associated genes was observed. Specifically, increases in the expression of COL3A1, COL5A1, DCN, FN1. FBN1, FBN2, ITGA2, LOX, LUM, SPARC, and CDC42 were observed. A decrease in MMP3 gene expression was also observed. MMP3 whose upregulation is associated with severe forms of RDEB and so decreasing its expression would potentially be beneficial to sufferers of that disease.

### Example 7: A study to evaluate ex-vivo the expression of collagen type VII protein in healthy human skin explant

### Methodology

Heat-treated collagenous jellyfish extracts were tested *ex vivo* on healthy skin explants NativeSkin^{®}, a full-thickness skin biopsy embedded in a solid and nourishing matrix while its epidermal surface is left in contact with air. The experiment was carried out on 5 donors (N=5) and explants were exposed to the test items for 5 days with heat-treated collagenous jellyfish extract.

After treatment, skin explants were fixed with formalin, embedded in paraffin and cut to allow the labelling. Collagen VII was labelled by immunofluorescence and observed by fluorescence microscopy (DM5000B - Leica Microsystems). The fluorescence intensity is measured by relative quantification compared to the control using Image J software. The means of % area of labelling at the level of basal layer measured on 10 pictures/condition ± standard error of mean (SEM) was represented, and the results obtained for products were compared to untreated condition.

### Results

The results of the immunofluorescent staining of fixed explants shown in Figure 8A indicate an increase in the level of collagen VII protein present in explants treated with heat-treated collagenous jellyfish extract compared to untreated controls. The increase seen in the treated explants was quantified and expressed as a percentage increase in area of labelling of collagen VII protein relative to the untreated control (Figure 8B).

### Example 8: An in vitro study to evaluate the gene expression of collagen type VII in healthy human keratinocyte (NHEK)

### Methodology

Healthy human epidermal keratinocytes (NHEK) were cultured with appropriate culture media containing heat-treated collagenous jellyfish extract. Treatment was carried out for 48 hours at 37°C. The expression of the COL7A1 mRNA was measured by semi-quantitative PCR on the Applied Biosystems 7300 Real Time PCR System. The expression level of the mRNAs calculated and normalised with reference gene (GAPDH). The values were reported to the untreated control so as to indicate an activating or inhibitory effect (n = 3) and error bars represent standard error of the mean.

### Results

The results shown in Figure 9 indicate that treatment of NHEK with heat-treated collagenous jellyfish extract leads to a significant increase in the expression of COL7A1 mRNA relative to an untreated control. This observation is consistent with the immunostaining data described in Example 7 above.

### Example 9: Ex-vivo study to evaluate jellyfish collagenous extract on the expression of collagen type VII, Keratin 5 and Laminin 5 in healthy human skin explant biopsies.

### Methodology

In this study the effect of heat-treated collagenous jellyfish extract on the protein expression of collagen 7, laminin 5, and keratin 5 in an *ex vivo* healthy human skin model. The study was carried out on skin explants NativeSkin^{®}, a full-thickness skin biopsy embedded in a solid and nourishing matrix while its epidermal surface is left in contact with air. The skin biopsy is firmly embedded in the matrix that prevents any lateral diffusion of topically applied formulations. The study was carried out on 10 donors (n=10) and explants were exposed to the test items for 5 days with heat-treated collagenous jellyfish extract.

After treatment, skin explants were fixed with formalin, embedded in paraffin and cut to allow the labelling. Biomarkers of interest were labelled by immunofluorescence and observed by fluorescence microscopy (DM5000B - Leica Microsystems). The fluorescence intensity is measured by relative quantification compared to the control using Image J software (Biomarkers quantified = collagen 7, keratin 5 and laminin 5).

The means of fluorescence intensity of protein labelling were measured on 10 pictures/condition and ± standard error of the mean (SEM) is represented. The results obtained for the heat-treated collagenous jellyfish extract were compared to untreated condition. Figure 10 shows representative images used in the immunostaining analysis.

### Results

The global results for all 10 donors in respect to laminin 5, collagen 7 and keratin 5 protein expression in *ex vivo* healthy human skin are shown in Tables 10, 11, and 12 below. Those results indicate that the expression of all the tested proteins is increased in explants treated with heat-treated collagenous jellyfish extract. An increase of almost 25% is observed for collagen 7, which is strongly implicated in the disease mechanism of a number of types of EB.

**Table 10: Summary of laminin 5 protein expression results in healthy human skin explants**

| **Value** | **Untreated control** | **Heat-treated collagenous jellyfish extract** |
|---|---|---|
| Fluorescence intensity of laminin 5 staining (%) | 100.0% | 104.3% |
| SEM | 1.2 | 1.6 |
| Significance relative to control | N/A | 0.01 < p < 0.05 |
| P-value | N/A | 0.03 |

**Table 11: Summary of collagen 7 protein expression results in healthy human skin explants**

| **Value** | **Untreated control** | **Heat-treated collagenous jellyfish extract** |
|---|---|---|
| Fluorescence intensity of collagen 7 staining (%) | 100.0% | 124.3% |
| SEM | 2.1 | 4.7 |
| Significance relative to control | N/A | p < 0.001 |
| P-value | N/A | 0.0001 |

**Table 12: Summary of keratin 5 protein expression results in healthy human skin explants**

| **Value** | **Untreated control** | **Heat-treated collagenous jellyfish extract** |
|---|---|---|
| Fluorescence intensity of keratin 5 staining (%) | 100.0% | 102.1% |
| SEM | 0.3 | 0.7 |
| Significance relative to control | N/A | 0.001 < p < 0.01 |
| P-value | N/A | 0.007 |

### Example 10: In vitro study to determine the effect of heat-treated jellyfish collagenous extract on gene activation and protein expression of Laminin-5, Collagen IV and Collagen VII in human diseased fibroblast cell lines of DEB, EBS and JEB.

### Experimental set-up

This study evaluated the effect of heat-treated collagenous jellyfish extract on the mRNA and protein expression of Collagen 7, Laminin 5 and Collagen 4 in EB-disease cell lines. The test was carried out on human fibroblasts from three donors suffering from EB.

### Donor 1 = D1 = male, 6 years, Simplex EB (SEB)

Epidermolysis bullosa simplex (EBS), is a disorder resulting from mutations in the genes encoding Keratin 5 or Keratin 14.

### Donor 2 = D2= female, 12 years, dystrophic EB (DEB)

Dystrophic epidermolysis bullosa (DEB) is a form of inherited epidermolysis bullosa (EB) characterised by cutaneous and mucosal fragility resulting in blisters and superficial ulcerations. DEB is caused by mutations in the COL7A1 (3p21.31) gene encoding the type VII collagen protein. Mutations in this gene alter the function, reduce or disrupt the production of collagen VII.

### Donor 3 = D3= male, 8 months, Junctional EB (JEB)

Junctional epidermolysis bullosa is a skin condition characterised by blister formation within the lamina lucida of the basement membrane zone. Junctional epidermolysis bullosa most commonly results from mutations in the LAMA3, LAMB3, LAMC2, and COL17A1 genes.

In order to allow comparison with healthy skin, an NHDF (Normal human dermal fibroblast), female, 20 years old, was used.

### mRNA analysis

The cells were treated with appropriate media with or without the test extracts and incubated for 48 hours at 37°C. After treatment, RNA was extracted from the cells and reverse-transcribed into cDNA. The expression of the COL4A1, COL7A1, LAMC2 mRNA was measured by semi-quantitative PCR on the Applied Biosystems 7300 Real Time PCR System. The expression level of the mRNAs was calculated and normalised with reference genes (GAPDH). The values were reported to the untreated control to express an activating or inhibitory effect and the results are expressed as an induction factor. The results are considered significant when the mRNA expression is +/- 40% (n = 3).

### Protein expression analysis

The cells were treated with appropriate media with or without the test extracts and incubated for 48 hours at 37°C. After treatment, the cells were washed and fixed with Formalin. After permeabilisation, the cells were incubated with an anti-collagen 4, anti-collagen 7 or anti-laminin5 antibody and a relevant fluorescently-labelled secondary antibody (ALEXA). Finally, the nuclei were labelled with DAPI.

The labelled proteins were observed and quantified by automated fluorescence microscopy Cellinsight CX7 High-Content Screening Platform (Thermofisher). The fluorescence intensity ± standard error of the mean (SEM) is represented (n = 3). Values were compared to the control. The significance is correlated with the p-value: ns=no significant, * p-value < 0.5, ** p-value < 0.01, *** p-value < 0.001.

### Results

The results of the mRNA and protein expression analysis is summarised in Tables 13, 14, and 15 below.

**Table 13: Effect of heat-treated collagenous jellyfish extract on the mRNA and protein expression in simplex EB (SEB) fibroblasts (Donor 1) (n = 3)**

| **Gene/protein** | **Gene expression** | **Protein expression** |
|---|---|---|
| Collagen 4 | + | - |
| Collagen 7 | **=** | + |
| Laminin 5 | **=** | - |

| | | |
|---|---|---|
| Representation of variation trend (mRNA +/- 40%; protein +/- 10%) | | |

**Table 14: Effect of heat-treated collagenous jellyfish extract on the mRNA and protein expression in dystrophic EB (DEB) fibroblasts (Donor 2) (n = 3)**

| **Gene/protein** | **Gene expression** | **Protein expression** |
|---|---|---|
| Collagen 4 | + | - |
| Collagen 7 | = | = |
| Laminin 5 | + | + |

| | | |
|---|---|---|
| Representation of variation trend (mRNA +/- 40%; protein +/- 10%) | | |

**Table 15: Effect of heat-treated collagenous jellyfish extract on the mRNA and protein expression in junctional EB (JEB) fibroblasts (Donor 3) (n = 3)**

| **Gene/protein** | **Gene expression** | **Protein expression** |
|---|---|---|
| Collagen 4 | + | = |
| Collagen 7 | = | + |
| Laminin 5 | = | + |

| | | |
|---|---|---|
| Representation of variation trend (mRNA +/- 40%; protein +/- 10%) | | |

The mRNA quantification shows that treatment with heat-treated collagenous jellyfish extract led to significant increases in COL4A1 gene expression in all types of EB fibroblasts tested. In addition, LAMC2 gene expression was significantly increased in DEB cells treated with heat-treated collagenous jellyfish extract.

In relation to protein expression, the immunostaining analysis indicates that SEB and JEB cells treated with heat-treated collagenous jellyfish extract had significant increases in the level of laminin 5 protein present. In addition, treated SEB and JEB cells showed increased levels of collagen 7 protein.

This study demonstrates new positive effects of heat-treated collagenous jellyfish extract on fibroblasts from EB skin on the basal lamina-related proteins collagen 4, collagen 7 and laminin 5.

## Claims

1. A collagenous marine invertebrate extract for use in the treatment of Epidermolysis Bullosa, wherein the source of the extract is jellyfish, wherein the collagenous extract comprises collagen in a hydrolysate form.

2. The collagenous marine invertebrate extract for use according to Claim 1, wherein the collagen in hydrolysate form is produced by heat treatment of the extract.

3. The collagenous marine invertebrate extract for use according to Claim 1 or Claim 2, wherein the Epidermylosis Bullosa to be treated is selected from Epidermolysis bullosa simplex (EBS), Junctional epidermolysis bullosa (JEB), Dystrophic epidermolysis bullosa (DEB), Epidermolysis bullosa acquisita (EBA), and/or Kindler syndrome (KS).

4. The collagenous marine invertebrate extract for use according to any one of Claims 1 to 3 wherein the collagenous marine invertebrate extract:
(a). supports improved dermal cohesion and basal layer structuring of cells via the activation of the genes COL7A1, LAMA5, KRT5;
(b) downregulates MMP-3 activity supporting better treatment outcomes for severe RDEB;
(c) supports improved cell adhesion, growth, migration, and differentiation via the activation of the genes FN1;
(d) promotes extracellular matrix synthesis, collagen fibre organisation and structuring via activation of the genes COL3A1, FBN1, FBN2, SPARC, and LUM;
(e). supports improved dermal cohesion and basal layer structuring of cells via the activation of the genes COL7A1, LAMA5 and CDC42;
(f). supports dermis renewal and protection via the activation of Epithelial Growth Factor (EGF), SIRT4 and SIRT6;
(g). supports improved dermal cellular junction structure via activation of the genes EPPK and LOR, and/or
(h) supports improved extracellular matrix structure by reducing MMP3 activation involved in EB blistering.

5. The collagenous marine invertebrate extract for use according to any one of Claims 1 to 4, wherein the jellyfish is from the class *Scyphozoa.*

6. The collagenous marine invertebrate extract for use according to Claim 5, wherein the jellyfish is one or more selected from the group comprising: *Rhizostomas pulmo, Rhopilema esculentum, Rhopilema nomadica, Stomolophus meleagris, Aurelia sp., Nemopilema nomurai, Cassiopea andromeda, Nemopilema nomurai, Chrysaora sp.,* or any combination thereof.

7. The collagenous marine invertebrate extract for use according to Claim 6, wherein the jellyfish is *Rhizostomas pulmo.*

8. The collagenous marine invertebrate extract for use according to any one of Claims 1 to 7, wherein the collagenous marine invertebrate extract is in the form of a solution, powder, sponge matrix, nano-fibre electrospun matrix, a hydrogel or in a lyophilised form.

9. The collagenous marine invertebrate extract for use according to any one of Claims 1 to 8, wherein the collagenous marine invertebrate extract is administered in combination with at least one growth factor, optionally wherein the at least one growth factor is Platelet Rich Plasma (PRP), Epithelial Growth Factor (EGF), Transforming Growth Factor-Beta (TGF-B, TGF-B2, TGF-B3), Hepatocyte Growth Factor (HGF), Keratinocyte Growth Factor (KGF), Granulocyte-Monocyte Colony Stimulating Growth Factor, Platelet Derived Growth Factor, Insulin-like Growth Factor 1 (IGF1), basic Fibroblast Growth Factor (bFGF), and/or Vascular Endothelial Growth Factor (VEGF).

10. The collagenous marine invertebrate extract for use according to any one of Claims 1 to 9, wherein the collagenous marine invertebrate extract is administered in combination with at least one antimicrobial compound, optionally wherein the at least one antimicrobial compound is nano silver, penicillin, ofloxacin, tetracycline, aminoglycosides and erythromycin, flucloxacillin, clarithromycin, doxycycline, gentamicin, metronidazole, co-amoxiclav, co-trimoxazole (in penicillin), ceftriaxone, piperacillin with tazobactam, clindamycin, ciprofloxacin, vancomycin, teicoplanin, linezolid, and/or the standard of care antimicrobial agent.

11. A pharmaceutical composition for use in the treatment of Epidermolysis Bullosa, wherein the pharmaceutical composition comprises the collagenous marine invertebrate extract defined in any one of Claims 1 to 10.

12. The pharmaceutical composition for use according to Claim 11, wherein the pharmaceutical composition further comprises at least one pharmaceutically acceptable compound.

13. The pharmaceutical composition for use according to Claim 12, wherein the pharmaceutically acceptable compound is one or more selected from the group comprising: a growth factor, an antimicrobial, an antibiotic, and/or another Epidermolysis Bullosa therapeutic.

14. The pharmaceutical composition for use according to any one of Claims 11 to 13, wherein the pharmaceutical composition is formulated in any form normally used for topical application.

15. The pharmaceutical composition for use according to Claim 14, wherein the pharmaceutical composition is formulated as a cream, ointment, mask, serum, milk, lotion, paste, foam, aerosol, stick, shampoo, conditioner, patch, hydroalcoholic or oily aqueous solution, an oil-in-water or water-in-oil or multiple emulsion, an aqueous or oily gel, a liquid, pasty or solid anhydrous product, an electrospun collagen nano-fibre matrix, a membrane and/or an oil dispersion in an aqueous phase using spherules, these spherules being polymeric nanoparticles such as nanospheres and nanocapsules or lipid vesicles of ionic and/or non-ionic type, more preferably an electrospun collagen nano-fibre matrix and/or a membrane.

## Patentansprüche

1. Kollagener Extrakt mariner Wirbelloser zur Verwendung bei der Behandlung von Epidermolysis Bullosa, wobei die Quelle des Extrakts eine Qualle ist, wobei der kollagene Extrakt Kollagen in einer Hydrolysatform umfasst.

2. Kollagener Extrakt mariner Wirbelloser zur Verwendung nach Anspruch 1, wobei das Kollagen in Hydrolysatform durch Wärmebehandlung des Extrakts hergestellt wird.

3. Kollagener Extrakt mariner Wirbelloser zur Verwendung nach Anspruch 1 oder Anspruch 2, wobei die zu behandelnde Epidermylosis Bullosa ausgewählt ist aus Epidermolysis bullosa simplex (EBS), Epidermolysis bullosa junctionalis (JEB), Epidermolysis bullosa dystrophica (DEB), Epidermolysis bullosa acquisita (EBA) und/oder Kindler-Syndrom (KS).

4. Kollagener Extrakt mariner Wirbelloser zur Verwendung nach irgendeinem der Ansprüche 1 bis 3, wobei der kollagene Extrakt mariner Wirbelloser:
(a) die verbesserte dermale Kohäsion und Basalschichtstrukturierung von Zellen über die Aktivierung der Gene COL7A1, LAMA5, KRT5 unterstützt;
(b) die MMP-3-Aktivität herunterreguliert, wodurch bessere Behandlungsergebnisse bei schwerer RDEB unterstützt werden;
(c) die verbesserte Zelladhäsion, das Wachstum, die Migration und die Differenzierung über die Aktivierung der Gene FN1 unterstützt;
(d) die Synthese der extrazellulären Matrix, die Organisation und Strukturierung der Kollagenfasern über die Aktivierung der Gene COL3A1, FBN1, FBN2, SPARC und LUM fördert;
(e) die verbesserte dermale Kohäsion und Basalschichtstrukturierung von Zellen über die Aktivierung der Gene COL7A1, LAMA5 und CDC42 unterstützt;
(f) die Erneuerung und den Schutz der Dermis über die Aktivierung des Epithelialen Wachstumsfaktors (EGF), SIRT4 und SIRT6 unterstützt;
(g) die verbesserte Struktur der dermalen Zellverbindungen über die Aktivierung der Gene EPPK und LOR unterstützt, und/oder
(h) die verbesserte Struktur der extrazellulären Matrix durch Reduzieren der MMP3-Aktivierung unterstützt, die an der EB-Blasenbildung beteiligt ist.

5. Kollagener Extrakt mariner Wirbelloser zur Verwendung nach irgendeinem der Ansprüche 1 bis 4, wobei die Qualle aus der Klasse der *Scyphozoa* stammt.

6. Kollagener Extrakt mariner Wirbelloser zur Verwendung nach Anspruch 5, wobei die Qualle eines oder mehrere ausgewählt aus der Gruppe ist, die Folgendes umfasst: *Rhizostomas pulmo, Rhopilema esculentum, Rhopilema nomadica, Stomolophus meleagris, Aurelia sp., Nemopilema nomurai, Cassiopea andromeda, Nemopilema nomurai, Chrysaora sp.* oder irgendeine Kombination davon.

7. Kollagener Extrakt mariner Wirbelloser zur Verwendung nach Anspruch 6, wobei die Qualle *Rhizostomas pulmo* ist.

8. Kollagener Extrakt mariner Wirbelloser zur Verwendung nach irgendeinem der Ansprüche 1 bis 7, wobei der kollagene Extrakt mariner Wirbelloser in Form einer Lösung, eines Pulvers, einer Schwamm-Matrix, einer elektrogesponnenen Nanofaser-Matrix, eines Hydrogels oder in einer lyophilisierten Form vorliegt.

9. Kollagener Extrakt mariner Wirbelloser zur Verwendung nach irgendeinem der Ansprüche 1 bis 8, wobei der kollagene Extrakt mariner Wirbelloser in Kombination mit mindestens einem Wachstumsfaktor verabreicht wird, gegebenenfalls wobei der mindestens eine Wachstumsfaktor Plättchenreiches Plasma (PRP), Epithelialer Wachstumsfaktor (EGF), Transformierender Wachstumsfaktor-Beta (TGF-B, TGF-B2, TGF-B3), Hepatozyten-Wachstumsfaktor (HGF), Keratinozyten-Wachstumsfaktor (KGF), Granulozyten-Monozyten-Koloniestimulierender Wachstumsfaktor, von Blutplättchen abgeleiteter Wachstumsfaktor, insulinähnlicher Wachstumsfaktor 1 (IGF1), basischer Fibroblasten-Wachstumsfaktor (bFGF) und/oder Vaskulärer Endothelialer Wachstumsfaktor (VEGF) ist.

10. Kollagener Extrakt mariner Wirbelloser zur Verwendung nach irgendeinem der Ansprüche 1 bis 9, wobei der kollagene Extrakt mariner Wirbelloser in Kombination mit mindestens einer antimikrobiellen Verbindung verabreicht wird, gegebenenfalls wobei die mindestens eine antimikrobielle Verbindung Nanosilber, Penicillin, Ofloxacin, Tetracyclin, Aminoglykoside und Erythromycin, Flucioxacillin, Clarithromycin, Doxycyclin, Gentamicin, Metronidazol, Co-Amoxiclav, Co-Trimoxazol (in Penicillin), Ceftriaxon, Piperacillin mit Tazobactam, Clindamycin, Ciprofloxacin, Vancomycin, Teicoplanin, Linezolid und/oder das antimikrobielle Mittel zur Standardbehandlung ist.

11. Pharmazeutische Zusammensetzung zur Verwendung bei der Behandlung von Epidermolysis Bullosa, wobei die pharmazeutische Zusammensetzung den in irgendeinem der Ansprüche 1 bis 10 definierten kollagenen Extrakt mariner Wirbelloser umfasst.

12. Pharmazeutische Zusammensetzung zur Verwendung nach Anspruch 11, wobei die pharmazeutische Zusammensetzung ferner mindestens eine pharmazeutisch annehmbare Verbindung umfasst.

13. Pharmazeutische Zusammensetzung zur Verwendung nach Anspruch 12, wobei die pharmazeutisch annehmbare Verbindung eines oder mehrere ausgewählt aus der Gruppe ist, die Folgendes umfasst: einen Wachstumsfaktor, ein Antimikrobikum, ein Antibiotikum und/oder ein anderes Therapeutikum für Epidermolysis Bullosa.

14. Pharmazeutische Zusammensetzung zur Verwendung nach irgendeinem der Ansprüche 11 bis 13, wobei die pharmazeutische Zusammensetzung in irgendeiner Form formuliert ist, die normalerweise zur topischen Anwendung verwendet wird.

15. Pharmazeutische Zusammensetzung zur Verwendung nach Anspruch 14, wobei die pharmazeutische Zusammensetzung als Creme, Salbe, Maske, Serum, Milch, Lotion, Paste, Schaum, Aerosol, Stift, Shampoo, Konditionierer, Pflaster, hydroalkoholische oder ölige wässrige Lösung, eine Öl-in-Wasser- oder Wasser-in-Öl- oder multiple Emulsion, ein wässriges oder öliges Gel, ein flüssiges, pastöses oder festes wasserfreies Produkt, eine elektrogesponnene Kollagen-Nanofaser-Matrix, eine Membran und/oder eine Öldispersion in einer wässrigen Phase unter Verwendung von Kügelchen ist, wobei diese Kügelchen polymere Nanopartikel, wie etwa Nanokügelchen und Nanokapseln, oder Lipidvesikel vom ionischen und/oder nichtionischen Typ sind, besonders bevorzugt eine elektrogesponnene Kollagen-Nanofaser-Matrix und/oder eine Membran.

## Revendications

1. Extrait collagénique d'invertébré marin à utiliser dans le traitement de l'épidermolyse bulleuse, dans lequel la source de l'extrait est la méduse, dans lequel l'extrait collagénique comprend du collagène sous forme d'hydrolysat.

2. Extrait collagénique d'invertébré marin à utiliser selon la revendication 1, dans lequel le collagène sous forme d'hydrolysat est produit par traitement thermique de l'extrait.

3. Extrait collagénique d'invertébré marin à utiliser selon la revendication 1 ou la revendication 2, dans lequel l'épidermolyse bulleuse à traiter est choisie parmi l'épidermolyse bulleuse simplex (EBS), l'épidermolyse bulleuse jonctionnelle (EBJ), l'épidermolyse bulleuse dystrophique (EBD), l'épidermolyse bulleuse acquise (EBA), et/ou le syndrome de Kindler (KS).

4. Extrait collagénique d'invertébré marin à utiliser selon l'une quelconque des revendications 1 à 3, dans lequel l'extrait collagénique d'invertébré marin :
(a) prend en charge la cohésion dermique et la structuration de couche basale améliorées de cellules par l'intermédiaire de l'activation des gènes COL7A1, LAMA5, KRT5 ;
(b) régule à la baisse l'activité de la MMP-3, ce qui permet d'obtenir de meilleurs résultats dans le traitement de la RDEB sévère ;
(c) prend en charge l'adhésion cellulaire, la croissance, la migration et la différenciation améliorées par l'intermédiaire de l'activation des gènes FN1 ;
(d) favorise la synthèse de matrice extracellulaire, l'organisation et la structuration des fibres de collagène par l'intermédiaire de l'activation des gènes COL3A1, FBN1, FBN2, SPARC et LUM ;
(e) prend en charge la cohésion dermique et la structuration de couche basale améliorées de cellules par l'intermédiaire de l'activation des gènes COL7A1, LAMAS et CDC42 ;
(f) prend en charge le renouvellement et la protection du derme par l'intermédiaire de l'activation du facteur de croissance épithéliale (EGF), du SIRT4 et du SIRT6 ;
(g) prend en charge la structure de jonction cellulaire dermique améliorée par l'intermédiaire de l'activation des gènes EPPK et LOR, et/ou
(h) prend en charge la structure de matrice extracellulaire améliorée en réduisant l'activation de MMP3 impliquée dans le cloquage d'EB.

5. Extrait collagénique d'invertébré marin à utiliser selon l'une quelconque des revendications 1 à 4, dans lequel la méduse est de la classe des *Scyphozoa.*

6. Extrait collagénique d'invertébré marin à utiliser selon la revendication 5, dans lequel la méduse est une ou plusieurs méduses choisies dans le groupe comprenant : la *Rhizostomas pulmo, la Rhopilema esculentum, la Rhopilema nomadica, la Stomolophus meleagris, l'Aurelia sp., la Nemopilema nomurai, la Cassiopea andromeda, la Nemopilema nomurai, la Chrysaora sp.,* ou toute combinaison de celles-ci.

7. Extrait collagénique d'invertébré marin à utiliser selon la revendication 6, dans lequel la méduse est la *Rhizostomas pulmo.*

8. Extrait collagénique d'invertébré marin à utiliser selon l'une quelconque des revendications 1 à 7, dans lequel l'extrait collagénique d'invertébré marin se présente sous la forme d'une solution, une poudre, une matrice spongieuse, une matrice de nanofibres électrofilées, un hydrogel ou sous une forme lyophilisée.

9. Extrait collagénique d'invertébré marin à utiliser selon l'une quelconque des revendications 1 à 8, dans lequel l'extrait collagénique d'invertébré marin est administré en combinaison avec au moins un facteur de croissance, éventuellement dans lequel l'au moins un facteur de croissance est le plasma riche en plaquettes (PRP), le facteur de croissance épithélial (EGF), le facteur de croissance transformant bêta (TGF-B, TGF-B2, TGF-B3), le facteur de croissance hépatocytaire (HGF), le facteur de croissance kératinocytaire (KGF), le facteur de croissance stimulant les colonies de granulocytes et de monocytes, le facteur de croissance dérivé des plaquettes, le facteur de croissance analogue à l'insuline 1 (IGF1), le facteur de croissance basique des fibroblastes (bFGF) et/ou le facteur de croissance endothélial vasculaire (VEGF).

10. Extrait collagénique d'invertébré marin à utiliser selon l'une quelconque des revendications 1 à 9, dans lequel l'extrait collagénique d'invertébré marin est administré en combinaison avec au moins un composé antimicrobien, éventuellement dans lequel l'au moins un composé antimicrobien est du nanoargent, de la pénicilline, de l'ofloxacine, de la tétracycline, des aminoglycosides et de l'érythromycine, de la flucloxacilline, de la clarithromycine, de la doxycycline, de la gentamicine, du métronidazole, du coamoxiclav, du co-trimoxazole (dans la pénicilline), de la ceftriaxone, de la pipéracilline avec tazobactam, de la clindamycine, de la ciprofloxacine, de la vancomycine, de la teicoplanine, du linézolide, et/ou de l'agent antimicrobien standard de soins.

11. Composition pharmaceutique à utiliser dans le traitement de l'épidermolyse bulleuse, dans laquelle la composition pharmaceutique comprend l'extrait collagénique d'invertébré marin défini dans l'une quelconque des revendications 1 à 10.

12. Composition pharmaceutique à utiliser selon la revendication 11, dans laquelle la composition pharmaceutique comprend en outre au moins un composé pharmaceutiquement acceptable.

13. Composition pharmaceutique à utiliser selon la revendication 12, dans laquelle le composé pharmaceutiquement acceptable est un ou plusieurs composés choisis dans le groupe comprenant : un facteur de croissance, un antimicrobien, un antibiotique, et/ou un autre agent thérapeutique épidermolyse bulleuse.

14. Composition pharmaceutique à utiliser selon l'une quelconque des revendications 11 à 13, dans laquelle la composition pharmaceutique est formulée sous n'importe quelle forme normalement utilisée pour une application topique.

15. Composition pharmaceutique à utiliser selon la revendication 14, dans laquelle la composition pharmaceutique est formulée sous forme de crème, de pommade, de masque, de sérum, de lait, de lotion, de pâte, de mousse, d'aérosol, de stick, de shampooing, de conditionneur, de patch, de solution aqueuse hydroalcoolique ou huileuse, d'émulsion huile dans eau ou eau dans huile ou d'émulsion multiple, de gel aqueux ou huileux, de produit anhydre liquide, pâteux ou solide, de matrice de nano-fibres de collagène électrofilées, de membrane et/ou de dispersion huileuse en phase aqueuse utilisant des sphérules, ces sphérules étant des nanoparticules polymériques telles que des nanosphères et des nanocapsules ou des vésicules lipidiques de type ionique et/ou non ionique, plus préférentiellement une matrice de nano-fibres de collagène électrofilées et/ou une membrane.
